(19) Europäisches Patentamt
European Patent Office
Office européen des brevets

(11) Veröffentlichungsnummer: **0 230 242**
**A2**

# (12) EUROPÄISCHE PATENTANMELDUNG

(21) Anmeldenummer: **87100275.4**

(22) Anmeldetag: **12.01.87**

(51) Int. Cl.⁴: **C07K 5/02**, C07K 5/06, C07D 401/12, C07D 333/24, C07D 233/64, A61K 31/38, A61K 31/44, A61K 31/415, A61K 37/02, A61K 37/64

Patentansprüche für folgenden Vertragsstaten: AT + ES + GR.

(30) Priorität: **17.01.86 DE 3601248**

(43) Veröffentlichungstag der Anmeldung: **29.07.87 Patentblatt 87/31**

(84) Benannte Vertragsstaaten: **AT BE CH DE ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Breipohl, Gerhard, Dr.**
**Geisenheimer Strasse 95**
**D-6000 Frankfurt am Main(DE)**
Erfinder: **Knolle, Jochen, Dr.**
**Höchster Strasse 21**
**D-6239 Kriftel(DE)**
Erfinder: **Wegmann, Helmut, Dr.**
**Klarastrasse 4**
**D-6238 Hofheim am Taunus(DE)**
Erfinder: **Ruppert, Dieter, Dr.**
**Patrizierhof 1**
**D-6233 Kelkheim (Taunus)(DE)**

## (54) Substituierte 4-Amino-3-hydroxy-buttersäure-Derivate, Verfahren zu deren Herstellung, diese enthaltende Mittel und ihre Verwendung.

(57) Die Erfindung betrifft Verbindungen der Formel

$$R^1\text{-A-B-NH-}\underset{\displaystyle |}{\overset{\displaystyle R^2}{}}\!\!\text{CH-}\overset{OH}{\text{CH}}\text{-}\overset{R^3}{\text{CH}}\text{-}\overset{O}{\overset{\|}{\text{C}}}\text{-}R^4$$

worin $R^1$ abwesend ist oder Wasserstoff, Alkyl oder Acyl, A einen Carbonsäurerest oder einen Aminosäurerest, B einen gegebenenfalls N-alkylierten Aminosäurerest, $R^2$ Wasserstoff, Alkyl, Alkylcycloalkyl, Arylniederalkyl oder Aryl, $R^3$ Wasserstoff, Alkyl oder Alkylniederalkyl und $R^4$ substituiertes Amino darstellen sowie deren Salze, Verfahren zu ihrer Herstellung, diese enthaltende pharmazeutische Präparate und deren Verwendung als Arzneimittel sowie Zwischenprodukte zur Herstellung dieser Verbindungen.

EP 0 230 242 A2

**Substituierte 4-Amino-3-hydroxybuttersäure-Derivate, Verfahren zu deren Herstellung, diese enthaltende Mittel und ihre Verwendung**

Aus der EP-A-152 255 und der EP-A-155 809 sind Tripeptid-Derivate und deren Verwendung als Renin-Inhibitoren bekannt.

Es wurden neue substituierte 4-Amino-3-hydroxybuttersäure-Derivate gefunden, die in vitro und in vivo hochwirksam das Enzym Renin hemmen.

Die Erfindung betrifft Verbindungen der Formel I

$$R^1-A-B-NH-\underset{\displaystyle |}{\overset{R^2}{CH}}-\overset{OH}{CH}-\overset{R^3}{CH}-\overset{O}{\overset{\|}{C}}-R^4 \qquad (I)$$

in welcher

$R^4$ $a_1$) einen Rest der Formel II oder III bedeutet,

$$-\underset{R^5}{N}-\overset{R^6}{CH}-[CH_2]_n-Ar \qquad (II)$$

$$-\underset{R^5}{N}-\overset{R^6}{CH}-CH=CH-[CH_2]_m-Ar \qquad (III)$$

worin $R^5$ für Wasserstoff oder $(C_1-C_7)$-Alkyl und

$R^6$ für Wasserstoff, $(C_1-C_7)$-Alkyl oder durch Hydroxy, $(C_1-C_5)$-Alkoxy, $(C_1-C_5)$-Alkylthio, Carboxy, $(C_1-C_5)$-Alkoxycarbonyl, Cl, Br, $(C_1-C_5)$-Alkylamino, Di-$(C_1-C_5)$-alkylamino, $(C_1-C_5)$-Alkoxycarbonylamino oder $(C_1-C_{15})$-Aralkyloxycarbonylamino monosubstituiertes $(C_1-C_7)$-Alkyl stehen,

m = 0, 1, 2, 3 oder 4 ist,

n = 1, 2, 3, 4, 5 oder 6 ist,

die Doppelbindung im Rest der Formel III im Falle n = 0 vorzugsweise die E-Konfiguration, andernfalls vorzugsweise die Z-Konfiguration aufweist und

Ar für $(C_6-C_{14})$-Aryl, das gegebenenfalls durch einen oder zwei gleiche oder verschiedene Reste aus der Reihe F, Cl, Br, J, Hydroxy, $(C_1-C_7)$-Alkoxy, $(C_1-C_7)$-Alkyl, $(C_1-C_7)$-Alkoxycarbonyl, Amino, $(C_1-C_7)$-Alkylamino, Di-$(C_1-C_7)$-Alkylamino, Carboxy, Carboxymethyloxy, Amino-$(C_1-C_7)$-alkyl, $(C_1-C_7)$-Alkylamino-$(C_1-C_7)$-alkyl, Di-$(C_1-C_7)$-alkylamino-$(C_1-C_7)$-alkyl, $(C_1-C_7)$-Alkoxycarbonylmethoxy, Carbamoyl, Sulfamoyl, $(C_1-C_7)$-Alkoxysulfonyl, Sulfo und Guanidinomethyl substituiert ist, oder für den Rest eines 5-oder 6-gliedrigen monocyclischen oder 9-oder 10-gliedrigen bicyclischen Heteroaromaten mit mindestens 1 C-Atom, 1 -3 N-Atomen und/oder 1 S-oder O-Atom als Ringglieder steht, wobei Ar auch über -O-gebunden sein kann,

$a_2$) einen über eine Peptidbundung verknüpften Rest einer Aminosäure aus der Reihe Phenylalanin, Histidin, Tyrosin, Tryptophan, Methionin, Leucin, Isoleucin, Asparagin, Asparaginsäure, $\beta$-2-Thienylalanin, $\beta$-3-Thienylalanin, 4-Chlorphenylalanin, Methioninsulfon, Methioninsulfoxid, 2-Pyridylalanin, 3-Pyridylalanin, Cyclohexylalanin, Cyclohexylglycin, im-Methylhistidin, O-Methyltyrosin, O-Benzyltyrosin, O-tert.-Butyltyrosin, Phenylglycin, 1-Naphthylalanin, 2-Naphthylalanin, 4-Nitrophenylalanin, 2-Fluorphenylalanin, 3-Fluorphenylalanin, 4-Fluorphenylalanin, Norleucin, 1,2,3,4-Tetrahydroisochinolin-3-carbonsäure, Homophenylalanin, DOPA, O-Dimethyldopa, 2-Amino-4-(2-thienyl)-buttersäure, 2-Amino-4-(3-thienyl)-buttersäure, 3-(2-Thienyl)-serin, (Z)-Dehydrophenylalanin und (E)-Dehydrophenylalanin, dessen C-terminale Carboxylgruppe frei ist, zur $CH_2OH$ reduziert oder

als Amid, welches einen C-Terminus der Formel $-NR^7R^8$ aufweist, vorliegt, bedeutet worin $R^7$ und $R^8$ gleich

oder verschieden sind und für
Wasserstoff,
$(C_1-C_{10})$-Alkyl, das gegebenenfalls durch Amino, $(C_1-C_5)$-Alkoxycarbonylamino, $(C_7-C_{15})$-Aralkylcarbonylamino, Heteroarylamino, worin der Rest des Heteroaromaten wie oben unter $(a_1)$ definiert ist, Hydroxy, $(C_1-C_5)$-Alkoxy, Sulfo, Carboxy oder $(C_1-C_5)$-Alkoxycarbonyl monosubstituiert ist,
$(C_6-C_{14})$-Aryl-$(C_1-C_4)$-alkyl, das gegebenenfalls im Arylteil durch Amino, Carboxy, $(C_1-C_5)$-Alkoxycarbonyl, Amino-$(C_1-C_5)$-alkyl, $(C_1-C_5)$-Alkoxycarbonylamino$(C_1-C_5)$-alkyl, $(C_7-C_{15})$-Aralkoxycarbonylamino-$(C_1-C_5)$-alkyl, $(C_1-C_5)$-Alkoxycarbonyl-$(C_1-C_5)$alkoxy, Carboxy-$(C_1-C_5)$-alkoxy oder Guanido-$(C_1-C_5)$-alkoxy monosubstituiert ist, oder
Heteroaryl-$(C_1-C_4)$-alkyl, wobei der Rest des Heteroaromaten wie Ar unter $(a_1)$ definiert ist, oder
für einen Rest der Formel IV steht,

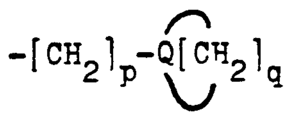

(IV)

worin p = 1, 2, 3 oder 4 ist, q = 4 oder 5 ist und Q N oder CH bedeutet oder p = 0 ist, q = 4 oder 5 ist und Q CH bedeutet und worin eine $CH_2$-Gruppe im Ring durch NH, O, S, CO, N-Bzl, N-Z oder N-BOC ersetzt sein kann, oder

$a_3)$ einen über eine Peptidbindung verknüpften Rest eines Dipeptids aus der Reihe Leu-Asn, Ile-His, Ile-Arg und Ile-Lys bedeutet, dessen C-Terminus als freie COOH-Gruppe, die gegebenenfalls zur $CH_2OH$-Gruppe reduziert ist, als $CONH_2$ oder als $COOR^9$ mit $R^9$ = $(C_1-C_7)$-Alkyl vorliegt und worin die $\epsilon$-Aminofunktion des Lys gegebenenfalls durch $(C_1-C_5)$-Alkoxycarbonyl oder $(C_7-C_{15})$-Aralkyloxycarbonyl geschützt ist;

$R^1$ $b_1)$ abwesend ist oder Wasserstoff bedeutet,
$b_2)$ $(C_1-C_{20})$-Alkyl, das gegebenenfalls durch ein, zwei oder drei gleiche oder verschiedene Reste aus der Reihe Hydroxy, $(C_1-C_7)$-Alkoxy, $(C_1-C_7)$-Alkanoyloxy, Carboxy, $(C_1-C_7)$-Alkoxycarbonyl, $(C_1-C_8)$-Alkanoyloxy, Cl, Br, Amino, $(C_1-C_7)$-Alkylamino, Di-$(C_1-C_7)$-alkylamino, $(C_1-C_5)$-Alkoxycarbonylamino oder $(C_7-C_{11})$-Aralkyloxycarbonylamino substituiert ist,
$(C_3-C_8)$-Cycloalkyl,
$(C_3-C_8)$-Cycloalkyl-$C_1-C_{10})$alkyl oder
$(C_6-C_{14})$-Aryl-$(C_1-C_8)$-alkyl, das im Arylteil gegebenenfalls durch einen oder zwei gleiche oder verschiedene Reste aus der Reihe F, Cl, Br, Hydroxy, $(C_1-C_7)$-Alkoxy, $(C_1-C_7)$-Alkyl, $(C_1-C_7)$-Alkoxycarbonyl, Amino oder Trifluormethyl substituiert ist, bedeutet oder
$b_3)$ einen Rest der Formel V bedeutet,
$R^a$-W-(V)
worin W für -CO-, -O-CO-, $-SO_2$-oder -NH-CO-steht und $R^a$ Wasserstoff, $(C_1-C_{10})$-Alkyl, das gegebenenfalls ein-oder zweifach ungesättigt ist und das gegebenenfalls durch bis zu 3 gleiche oder verschiedene Rest aus der Reihe Hydroxy, $(C_1-C_7)$-Alkoxy, $(C_1-C_7)$-Alkanoyloxy, Carboxy, $(C_1-C_7)$-Alkoxycarbonyl, Cl, Br, Amino, $(C_1-C_7)$-Alkylamino, Di-$(C_1-C_7)$-Alkylamino, $(C_1-C_5)$-Alkoxycarbonylamino, $(C_7-C_{15})$-Aralkoxycarbonylamino oder 9-Fluorenylmethyloxycarbonylamino substituiert ist,
$(C_3-C_8)$-Cycloalkyl,
$(C_3-C_8)$-Cycloalkyl-$(C_1-C_6)$alkyl, $(C_6-C_{14})$-Aryl, das gegebenenfalls durch einen oder zwei gleiche oder verschiedene Reste aus der Reihe F, Cl, Br, J, Hydroxy, $(C_1-C_7)$-Alkoxy, $(C_1-C_7)$-Alkyl, $(C_1-C_7)$-Alkoxycarbonyl, Amino, gegebenenfalls mit bis zu 2 Halogen substituiertes Anilino und Trifluormethyl substituiert ist,
$(C_6-C_{14})$-Aryl-$(C_1-C_6)$-alkyl, worin der Arylteil wie vorstehend bei Aryl definiert ist,
Heteroaryl oder Heteroaryl-$(C_1-C_6)$-alkyl, wobei der Rest des Heteroaromaten jeweils wie Ar unter $(a_1)$ definiert ist, bedeutet, oder
$b_4)$ einen Rest aus der Reihe (2S)-trans-4-Hydroxy-1-acyl-pyrrolidinyl-2-carbonyl, 5-Pyrrolidon-2S-carbonyl, $\alpha$-Methoxycarbonyl-$\alpha$-acylacetyl, 1-Acyl-(2R, 3aR, 6aR)-octahydro-cyclopenta[b]pyrrol-2-carbonyl und 1-Acyl-(2S,3aS,6aS)-octahydro-cyclopenta[a]pyrrol-2-carbonyl bedeutet, wobei Acyl die Bedeutung $(C_1-C_6)$-Alkanoyl, $(C_1-C_5)$-Alkoxycarbonyl oder $(C_7-C_{15})$-Alkoxycarbonyl oder $(C_7-C_{15})$-Aralkyloxycarbonyl hat,
A $c_1)$ einen Rest der Formeln VI oder VII bedeutet,

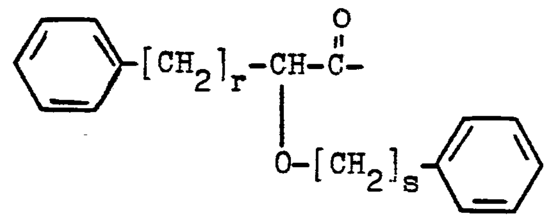

(VI)

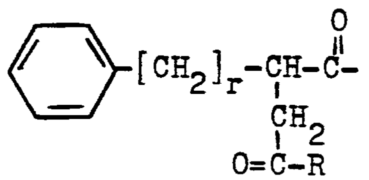

(VII)

in welchen
r = 1, 2 oder 3 ist,
s = 1, 2 oder 3 ist und
R ($C_1$-$C_7$)-Alkyl bedeutet, oder
$c_2$) einen N-terminal mit $R^1$ und C-terminal mit B verknüpften Rest einer Aminosäure bedeutet, die wie unter ($a_2$) definiert ist,
B $d_1$) falls $R^4$ wie unter ($a_1$) definiert ist, einen N-terminal mit A und C-terminal mit der NH-Gruppe von Formel I verknüpftem Rest einer Aminosäure bedeutet, die wie unter ($a_2$) definiert ist, oder
$d_2$) falls $R^4$ wie unter ($a_2$) oder ($a_3$) definiert ist, einen N-terminal mit A und C-terminal mit der NH-Gruppe von Formel I verknüpften Rest einer Aminosäure aus der Reihe 2-Thienylalanin, 3-Thienylalanin, Cyclohexylglycin, Phenylglycin, Asparaginsäure, Asparagin, Glutaminsäure, Glutamin und Methioninsulfon bedeutet,
$R^2$ Wasserstoff, ($C_1$-$C_{10}$)-Alkyl, ($C_4$-$C_7$)-Cycloalkyl, ($C_4$-$C_7$)-Cycloalkyl-($C_1$-$C_4$)-alkyl, ($C_6$-$C_{14}$)-Aryl oder ($C_6$-$C_{14}$)-Aryl-($C_1$-$C_4$)-alkyl bedeutet und
$R^3$ Wasserstoff, ($C_1$-$C_{10}$)-Alkyl, ($C_6$-$C_{14}$)-Aryl oder ($C_6$-$C_{14}$)-Aryl-($C_1$-$C_4$)-alkyl bedeutet,
sowie deren physiologisch verträglichen Salze.

Die durch $R^2$, Hydroxy und $R^3$ substituierten C-Atome können jeweils die R-, S-oder R,S-Konfiguration besitzen.

Alkyl kann geradkettig oder verzweigt sein. Entsprechendes gilt für davon abgeleitete Reste, wie z.B. Alkoxy, Alkylthio, Alkylamino, Dialkylamino, Alkanoyl und Aralkyl.

Unter Cycloalkyl werden auch alkylsubstituierte Reste, wie z.B. 4-Methylcyclohexyl oder 2,3-Dimethylcyclopentyl verstanden.

($C_6$-$C_{14}$)-Aryl ist beispielsweise Phenyl, Naphthyl, Biphenylyl oder Fluorenyl; bevorzugt ist Phenyl. Entsprechendes gilt für davon abgeleitete Reste, wie z.B. Aryloxy, Aroyl, Aralkyl und Aralkyloxy. Bevorzugte Aralkylreste sind Benzyl und Phenethyl.

Unter einem Rest eines 5-oder 6-gliedrigen monocyclischen oder 9-oder 10-gliedrigen bicyclischen Heteroaromaten mit mindestens 1 C-Atom, 1 -3 N-Atomen und/oder 1 S-oder O-Atom als Ringglieder werden Reste von Heteroaromaten verstanden, wie sie beispielsweise in Katritzky, Lagowski, Chemie der Heterocyclen, Berlin, Heidelberg 1968, Seite 3 -5 definiert sind. Monocyclische Heteroaromaten sind z.B. Thiophen, Furan, Pyrrol, Imidazol, Pyrazol, Pyridin, Pyrazin, Pyrimidin, Pyridazin, 1,2,4-Triazol, Thiazol, Isothiazol, Oxazol und Isoxazol. Bicyclische Heteroaromaten sind z.B. Benzothiophen, Benzofuran, Indol, Isoindol, Indazol, Benzimidazol, Chinolin, Isochinolin, Phthalazin, Chinoxalin, Chinazolin und Cinnolin. Entsprechendes gilt für von Heteroaryl abgeleitete Reste, wie z.B. Heteroaryloxy, Heteroaryl und Heteroarylalkyl.

Die Aminosäuren A und B in Formel I sind durch eine Amidbindung miteinander verknüpft, es handelt sich um natürliche oder nichtnatürliche α-Aminosäuren der L-, D-oder D,L-Konfiguration, vorzugsweise der L-Konfiguration. Entsprechendes gilt für Aminosäuren oder deren Derivate als Reste $R^4$.

Unter Salzen von Verbindungen der Formel I sind insbesondere pharmazeutisch verwendbare oder nicht-toxische Salze zu verstehen.

Solche Salze werden beispielsweise von Verbindungen der Formel I, welche saure Gruppen, z.B. Carboxy, enthalten, mit Alkali-oder Erdalkalimetallen gebildet, wie Na, K, Mg und Ca, sowie mit physiologisch verträglichen organischen Aminen, wie z.B. Triethylamin und Tri-(2-hydroxy)-ethyl)-amin.

Verbindungen der Formel I, welche basische Gruppen, z.B. eine Aminogruppe oder eine Guanidinogruppe, enthalten, bilden Salze mit anorganischen Säuren, wie z.B. Salzsäure, Schwefelsäure oder Phosphorsäure und mit organischen Carbon-oder Sulfonsäuren,wie z.B. Essigsäure, Citronensäure, Benzoesäure, Maleinsäure, Fumarsäure, Weinsäure und p-Toluolsulfonsäure.

Bevorzugt sind Verbindungen der Formel I, in welcher $R^4$ wie oben unter ($a_1$) definiert ist. Besonders bevorzugt sind Verbindungen, in welchen $R^5$ Wasserstoff oder Methyl und/oder $R^6$ Wasserstoff, Methyl, Ethyl, Propyl, Isopropyl, Butyl, Isobutyl, sec. Butyl, ($C_1$-$C_5$)-Alkoxycarbonylmethyl, -($C_1$-$C_5$)-Alkoxycarbonylethyl oder Methylthioethyl bedeuten und/oder worin Ar für Phenoxy, Phenyl , 2-, 3-oder 4-Pyridyl, 4-Imidazolyl, 1-, 3-oder 4-Isochinolyl, 2-, 3-oder 4-Carboxyphenyl, 2-, 3-oder 4-($C_1$-$C_7$)-Alkoxycarbonylphenyl, vorzugsweise 3-Methoxycarbonylphenyl oder 3-tert.-Butoxycarbonylphenyl, 2-, 3-oder 4-Aminophenyl, Mono-oder Di-($C_1$-$C_7$)-alkylaminophenyl, vorzugsweise 3-Methylaminophenyl oder 3-Dimethylaminophenyl, 2-, 3-oder 4-Carbamoylphenyl, 3-Sulfamoylphenyl, 3-Sulfophenyl, Amino-($C_1$-$C_7$)-alkylphenyl, vorzugsweise 2-, 3-oder 4-Aminomethylphenyl, 3-(Methylamino-methyl)-phenyl, 3-(N,N-Dimethylaminomethyl)-phenyl, ($C_1$-$C_7$)-Alkoxycarbonylmethoxyphenyl, vorzugsweise 2-, 3-oder 4-Methoxycarbonylmethoxyphenyl oder 3-tert.-Butoxycarbonylmethoxyphenyl, 2-, 3-oder 4-Carboxymethyloxyphenyl, ($C_1$-$C_7$)-Alkoxyphenyl, vorzugsweise 2-, 3-oder 4-Methoxyphenyl, oder 2-, 3-oder 4-Guanidinomethylphenyl steht.

Von den Verbindungen der Formel I, in welcher $R^4$ wie oben unter ($a_2$) definiert ist, sind solche bevorzugt, in der $R^4$ für den Rest eines Aminosäureamids mit dem C-Terminus -$NR^7R^8$ steht, worin $R^7$ und $R^8$ gleich oder verschieden sind und Methyl, 10-Aminodecyl, 9-Aminononyl, 8-Aminooctyl, 7-Aminoheptyl, 6-Aminohexyl, 5-Aminopentyl, 4-Aminobutyl oder die N-BOC-oder N-Z-geschützten Derivate der genannten $\omega$-Aminoalkylreste, (2-Pyrimidinyl-amino)-ethyl, 2-Sulfoethyl, 8-Carboxoctyl, 7-Carboxyheptyl, 6-Carboxylhexyl, 7-Methoxycarbonyl-heptyl, 2-Pyridylaminoethyl, Phenethyl oder Benzyl, die beide gegebenenfalls im Phenylteil in 2-, 3-oder 4-Position durch Amino, in 2-, 3-oder 4-Position durch Aminomethyl, in 3-Position durch BOC-Aminomethyl oder Guanidinomethyl, in 2-, 3-oder 4-Position durch Carboxy, in 3-Position durch tert.-Butoxycarbonyl, in 2-, 3-oder 4-Position durch Carboxymethoxy oder in 3-Position durch tert.-Butoxycarbonylmethoxy substituiert sind, 2-, 3-oder 4-Pyridylmethyl, 4-Imidazolylethyl, 3-Indolylethyl, 4-Piperidyl, 4-Piperidyl-($C_1$-$C_4$)-alkyl, 2-Oxo-1-pyrroldinyl, 2-Oxo-1-pyrrolidinyl-($C_1$-$C_4$)-alkyl bedeuten, vorzugsweise 4-(N-Benzyl)-piperidyl, 3-Morpholinopropyl oder 3-(2-Oxo-1-pyrrolidinyl)-propyl bedeuten oder $NR^7R^8$ für Morpholino steht.

Verbindungen der Formel I mit einem von oben unter ($a_3$) definierten Rest $R^4$ sind vorzugsweise solche, worin $R^4$ für Leu-Asn-$NH_2$, Ile-His-OH, Ile-His-$NH_2$, Ile Arg-$NH_2$, Ile-Arg-OH, Ile-Arg-$OCH_3$, Ile-Arginol, Ile-Lys-(Boc)-$OCH_3$, Ile-Lys-$OCH_3$, Ile-Lys-OH oder Ile-Lys-$NH_2$ steht.

$R^1$ ist vorzugsweise abwesend, bedeutet Wasserstoff oder steht für ($C_1$-$C_{10}$)-Alkyl, Cyclopentyl, Cyclohexyl, Cyclopentyl-($C_1$-$C_{10}$)-alkyl, Cyclohexyl-($C_1$-$C_{10}$)-alkyl, gegebenenfalls substituiertes Phenyl-($C_1$-$C_8$)-alkyl, $H_2N$-($C_1$-$C_{10}$)-Alkyl, HO-($C_1$-$C_{10}$)-Alkyl, ($C_1$-$C_4$)-Alkoxy-($C_1$-$C_{10}$)-alkyl, ($C_1$-$C_4$)-Alkoxycarbonyl-($C_1$-$C_{10}$)-alkyl, Carboxy-($C_1$-$C_{10}$)-alkyl, ($C_1$-$C_8$)-Alkanoyloxy-($C_1$-$C_{10}$)-alkyl, ($C_1$-$C_{11}$)-Alkanoyl, wie n-Decanoyl, Formyl, Acetyl, Pivaloyl, Isovaleryl oder Isobutyryl, gegebenenfalls geschütztes Amino-($C_1$-$C_{11}$)-alkanoyl, wie 4-Aminobutyryl, 5-Aminopentanoyl, 6-Aminohexanoyl, 4-N-tert.-Butoxycarbonylaminobutyryl, 5-N-tert.-Butoxycarbonylaminopentanoyl oder 6-N-tert.-Butoxycarbonylaminohexanoyl, Di-($C_1$-$C_7$)-alkylamino-($C_1$-$C_{11}$)-alkanoyl, wie Dimethylaminoacetyl, ($C_4$-$C_9$)-Cycloalkanoyl, wie Cyclopropanoyl, Cyclobutanoyl, Cyclopentanoyl oder Cyclohexanoyl, ($C_6$-$C_{10}$)-Aryl-($C_1$-$C_{11}$)-alkanoyl, wie Phenylacetyl, Phenylpropanoyl oder Phenylbutanoyl, 2-(o,o-Dichloroanilino)-phenylacetyl, 2-(N-Benzyl-o,o-dichloranilino)-phenylacetyl, gegebenenfalls durch Halogen, ($C_1$-$C_7$)-Alkyl, ($C_1$-$C_7$)-Alkoxy oder ($C_1$-$C_7$)-Alkoxycarbonyl substituiertes Benzoyl, wie 4-Chlorbenzoyl, 4-Methylbenzoyl, 2-Methoxycarbonylbenzoyl oder 4-Methoxybenzoyl, Pyrrolyl-2-carbonyl, Pyridyl-3-carbonyl, Benzylsulfonyl, ($C_1$-$C_{10}$)-Alkoxycarbonyl, wie Methoxycarbonyl oder tert.-Butoxycarbonyl, durch Halogen substituiertes ($C_1$-$C_{10}$)-Alkoxycarbonyl, wie 2,2,2-Trichlorethoxycarbonyl oder 1,1-Dimethyl-2,2,2-trichlorethoxycarbonyl, ($C_6$-$C_{14}$)-Aryl-($C_1$-$C_6$)-alkoxycarbonyl, wie Benzyloxycarbonyl oder 9-Fluorenylmethylcarbonyl, (2S)-trans-4-Hydroxy-1-acetyl-pyrrolidinyl-2-carbonyl, $\alpha$-Methoxycarbonyl-$\alpha$-benzyloxy-carbonylaminoacetyl, N-Acetyl-(2S,3aS,6aS)-octahydrocyclopenta[b]pyrrol-2-carbonyl oder N-Acetyl-(2R,3aR,6aR)-octahydrocyclopenta[b]pyrrol-2-carbonyl.

Bevorzugte Aminosäuren, die für die Reste A, B und $R^4$ in Frage kommen, sind Phenylalanin, Histidin, Tyrosin, Tryptophan, Methionin, Leucin, Isoleucin, Asparagin, Asparaginsäure, $\beta$-2-Thienylalanin, $\beta$-3-Thienylalanin, 4-Chlorphenylalanin, Methioninsulfon, Methioninsulfoxid, 2-Pyridylalanin, 3-Pyridylalanin, Cyclohexylalanin, Cyclohexylglycin, im-Methylhistidin, O-Methyltyrosin, O-Benzyltyrosin, O-tert.-Butyltyrosin, Phenylglycin, 1-Naphthylalanin, 2-Naphtylalanin, 4-Nitrophenylalanin, Norleucin, 1,2,3,4-Tetrahydroisochinolin-3-carbonsäure, Homophenylalanin, 2-Amino-4-(2-thienyl)-buttersäure, (Z)-Dehydrophenylalanin oder (E)-Dehydrophenylalanin. Daneben bedeutet A vorzugsweise (2S)-Benzyloxy-3-phenyl-propanoyl oder (2R,S)-2-Benzyl-4-oxo-6,6-dimethyl-heptanoyl.

$R^2$ bedeutet vorzugsweise Cyclohexylmethyl, Benzyl oder Isobutyl, $R^3$ vorzugsweise Wasserstoff, Ethyl, sec.-Butyl, Isobutyl, Benzyl, Phenethyl oder 3-Phenylpropyl.

Ohne daß die Erfindung darauf beschränkt wäre, seien die nachstehenden erfindungsgemäßen Verbindungen hervorgehoben:

BOC-Phe-His-ACHPA-(1S)-1-methyl-3-phenylpropylamid
BOC-Phe-β-Thienylalanin-ACHPA-(1S)-1-methyl-3-phenylpropylamid
BOC-Phe-β-2-Thienylalanin-ACHPA-(1S)-1-methyl-3-(2-pyridyl)-propylamid
BOC-Phe-β-2-Thienylalanin-Sta-Ile-2-pyridylmethylamid
BOC-Phe-β-2-Thienylalanin-ACHPA-(1S)-1-methyl-3-(3-aminomethyl)-phenyl-propylamid
BOC-Phe-β-2-Thienylalanin-ACHPA-(1S)-1-methyl-3-(3-carboxymethoxy)-phenylpropylamid
BOC-Phe-β-2-Thienylalanin-ACHPA-(1S)-1-methyl-4-(3-carboxymethoxy)-phenylbutylamid
BOC-Phe-β-2-Thienylalanin-ACHPA-[(3S,4S)-4-methyl-hexan-3-yl]-amid
BOC-Phe-β-2-Thienylalanin-Sta-Ile-(3-aminomethyl)-benzylamid
Z-Phe-β-2-Thienylalanin-Sta-Ile-(3-carboxymethoxy)-benzylamid
Fmoc-Phe-β-2-Thienylalanin-Sta-Ile-(3-aminomethyl)-benzylamid
BOC-β-2-Thienylalanin-His-Sta-Ile-2-pyridylmethylamid
Z-Phe-β-2-Thienylalanin-Sta-Ile-B-aminooctylamid
Z-Phe-β-2-Thienylalanin-Sta-Ile-(3-aminomethyl)-benzylamid
Z-Phe-β-2-Thienylalanin-ACHPA-Ile-(3-aminomethyl)-benzylamid
Z-Phe-His-ACHPA-(1S)-1-methyl-3-(2-pyridyl)-propylamid
Z-Phe-His-ACHPA-(1S)-1-methyl-3-(3-aminomethyl)-phenylpropylamid
BOC-Phe-His-Sta-Leu-Asn-$NH_2$
BOC-Phe-β-2-Thienylalanin-Sta-Ile-Arg-OH
BOC-Phe-β-2-Thienylalanin-Sta-Ile-Arg-OMe
BOC-Phe-β-2-Thienylalanin-Sta-Ile-Arginol
BOC-Phe-β-2-Thienylalanin-Sta-Ile-Arg-$NH_2$

Die Erfindung betrifft weiterhin ein Verfahren zur Herstellung von Verbindungen der Formel I, das dadurch gekennzeichnet ist, daß man ein Fragment mit endständiger Carboxylgruppe oder dessen reaktives Derivat mit einem entsprechenden Fragment mit freier Aminogruppe kuppelt, gegebenenfalls zum Schutz weiterer funktioneller Gruppen (eine) temporär eingeführte Schutzgruppe(n) abspaltet und die so erhaltene Verbindung gegebenenfalls in ihr physiologisch verträgliches Salz überführt.

Fragmente einer Verbindung der Formel I mit einer endständigen Carboxylgruppe besitzen die nachstehenden Formeln VIIIa -VIIId:

$R^1$-OH (VIIIa)
$R^1$-A-OH (VIIIb)
$R^1$-A-B-OH (VIIIc)

$$R^1\text{-A-B-NH-}\underset{}{\overset{R^2}{\overset{|}{C}}}H\text{-}\overset{OH}{\overset{|}{C}}H\text{-}\overset{R^3}{\overset{|}{C}}H\text{-}\overset{O}{\overset{\|}{C}}\text{-OH} \qquad (VIIId)$$

Fragmente einer Verbindung der Formel I mit einer endständigen Aminogruppe besitzen die nachstehenden Formeln IXa -IXd:

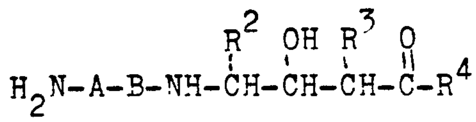
(IXa)

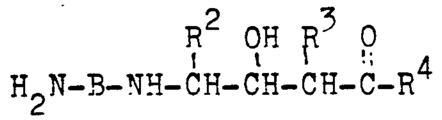
(IXb)

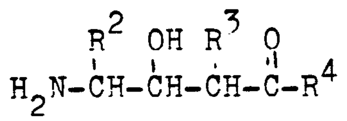
(IXc)

$H_2N-R^4$ (IXd)

Methoden, die zur Herstellung einer Amidbindung geeignet sind, werden z.B. in Houben-Weyl, Methoden der organischen Chemie, Band 15/2 beschrieben, vorzugsweise werden die folgenden Methoden herangezogen: Aktivestermethode mit N-Hydroxy-succinmid als Esterkomponente, Kopplung mit Propanphosphonsäureanhydrid und die Gemischt-Anhydrid-Methode mit Pivaloylchlorid.

Die Herstellung der als Ausgangsverbindung verwendeten optisch aktiven Amine der Formeln X und XI

$$R^5-HN-CH-[CH_2]_n-Ar \qquad (X)$$

$$R^5-HN-CH-CH=CH-[CH_2]_m-Ar \qquad (XI)$$

worin m, n, $R^5$, $R^6$ und Ar wie oben definiert sind, erfolgt ausgehend von optisch aktiven $\alpha$-Aminosäuren, wobei deren Asymmetriezentrum erhalten bleibt. Hierzu wird in bekannter Weise ein N-geschützter Aminosäurealdehyd hergestellt, welcher durch eine Wittig-Reaktion mit einem entsprechenden Phosphoniumsalz ein Olefin der Formel XI ergibt. Amine der Formel X werden daraus durch katalytische Hydrierung erhalten. Die Überprüfung der optischen Reinheit der hergestellten Amine kann in bekannter Weise durch Überführung in Mosher-Derivate durchgeführt werden (H.S. Mosher et. al. J. org. Chem. 34, 2543 (1969)).

Die Herstellung N-geschützter Aminosäurealdehyde erfolgt nach B. Castro et. al. (Synthesis 1983, 676).

Die Wittig-Reaktion eines N-geschützten Aminosäurealdehydes (bevorzugterweise N-tert.-Butoxycarbonyl-und Benzyloxycarbonyl Schutzgruppen) erfolgt in einem gegenüber geeigneten Basen inerten Lösungsmittel, wie Ether, THF, Toluol, DMF, DMSO oder Dimethoxyethan. Als Phosphoniumsalze werden vorzugsweise Alkyl-, Arylalkyl-und Heteroarylalkyl-Triphenylphosphonium-Chloride, -Bromide und -Jodide verwendet. Als Basen zur Deprotonierung der Phosphonium-Salze können Alkalimetallalkoholate, wie Kalium-tert.-butylat, Natriummethylat, Alkalimetallhydride, wie Natrium-oder Kaliumhydrid, metallorganische Basen, wie n-Butyllithium, s-Butyllithium, Methyllithium oder Phenyllithium, Natriumamid sowie Alkalimetallsalze von organischen Stickstoffbasen, wie Lithiumdiisopropylamid verwendet werden.

Die in den erfindungsgemäßen Verbindungen der allgemeinen Formel I enthaltenen substituierten 4-Amino-3-hydroxy buttersäuren sind literaturbekannt und werden nach D.H. Rich et. al. J. Org. Chem. 43, 3624 (1978) hergestellt. Die zur Herstellung von Verbindungen der Formel I erforderlichen Vor-und Nachoperationen wie Einführung und Abspaltung von Schutzgruppen sind literaturbekannt und sind z.B. in T.W. Greene, "Protective Groups in Organic Synthesis" beschrieben. Salze von Verbindungen der Formel I mit salzbildenden Gruppen werden in an sich bekannter Weise hergestellt, in dem man z.B. eine Verbindung der Formel I mit einer basischen Gruppe mit einer stöchiometrischen Menge einer geeigneten Säure umsetzt. Stereoisomerengemische, insbesondere Diastereomerengemische, die bei Verwendung racemischer Aminosäuren A oder B anfallen, können in an sich bekannter Weise, beispielsweise durch fraktionierte Kristallisation oder durch Chromatographie getrennt werden. Die erfindungsgemäßen Verbindungen der Formel I weisen enzymhemmende Eigenschaften auf; insbesondere hemmen sie die Wirkung des natürlichen Enzyms Renin. Renin ist ein proteolytisches Enzym aus der Klasse der Aspartyl-Proteasen welches als Folge verschiedener Stimuli (Volumendepletion, Natriummangel, $\beta$-Rezeptorenstimulation) von den juxtaglomerulären Zellen der Niere in den Blutkreislauf sezerniert wird. Dort spaltet es von dem aus der Leber ausgeschiedenem Angiotensinogen das Decapeptid Angiotensin 1 ab. Dieses wird durch das

"angiotensin converting enzyme" (ACE) in Angiotensin 2 überführt. Angiotension 2 spielt eine wesentliche Rolle bei der Blutregulation, da es direkt den Blutdruck durch Gefäßkontraktion steigert. Zusätzlich stimuliert es die Sekretion von Aldosteron aus der Nebenniere und erhöht auf diese Weise über die Hemmung der Natrium-Ausscheidung das extrazelluläre Flüssigkeitsvolumen, was seinerseits zu einer Blutdrucksteigerung beiträgt. Hemmer der enzymatischen Aktivität des Renins bewirken eine verminderte Bildung von Angiotensin 1, was eine verminderte Bildung von Angiotensin 2 zur Folge hat. Die Erniedrigung der Konzentration dieses aktiven Peptidhormons ist die direkte Ursache für die blutdrucksenkende Wirkung von Renin-Hemmern.

Die Wirksamkeit von Renin-Hemmern kann durch in-vitro-Tests überprüft werden. Hierbei wird die Verminderung der Bildung von Angiotensin 1 in verschiedenen Systemen (Humanplasma, Schweine-Renin) gemessen. Hierzu wird z.B. Humanplasma, welches sowohl Renin als auch Angiotensinogen enthält, bei 37°C mit der zu testenden Verbindung inkubiert. Anschließend wird die Konzentration des während der Inkubation gebildeten Angiotensin 1 mit einem Radioimmunoessay gemessen. Die in der vorliegenden Erfindung beschriebenen Verbindungen der allgemeinen Formel I zeigen in den verwendeten in-vitro-Tests Hemmwirkungen bei Konzentrationen von etwa $10^{-5}$ bis $10^{-10}$ Mol/1.

Renin-Hemmer bewirken an salzverarmten Tieren eine Blutdrucksenkung. Da sich menschliches Renin von dem Renin anderer Spezies unterscheidet werden zum in-vivo-Test von Renin-Hemmern Primaten - (Marmosets, Rhesus-Affen) herangezogen. Primaten-Renin und Human-Renin sind in ihrer Sequenz weitgehend homolog. Durch i.v. Injektion von Furosemid wird eine endogene Renin-Ausschüttung angeregt. Anschließend werden die Testverbindungen durch kontinuierliche Infusion verabreicht und ihre Wirkung auf Blutdruck und Herzfrequenz wird gemessen. Die Verbindungen der vorliegenden Erfindung sind hierbei in einem Dosisbereich von etwa 0,1 -5 mg/kg i.v. wirksam. Die in der vorliegenden Erfindung beschriebenen Verbindungen der allgemeinen Formel I können als Antihypertensiva, sowie zur Behandlung der Herzinsuffizienz verwendet werden.

Die Erfindung betrifft daher auch die Verwendung von Verbindungen der Formel I als Heilmittel und pharmazeutische Präparate, die diese Verbindungen enthalten. Bevorzugt ist die Anwendung beim Menschen.

Pharmazeutische Präparate enthalten eine wirksame Menge des Wirkstoffs der Formel I zusammen mit einem anorganischem oder organischen pharmazeutisch verwendbaren Trägerstoff enthalten. Die Anwendung kann intranasal, intravenös, subcutan oder peroral erfolgen. Die Dosierung des Wirkstoffs hängt von der Warmblüter-Spezies, dem Körpergewicht, Alter und von der Applikationsart ab.

Die pharmazeutischen Präparate der vorliegenden Erfindung werden in an sich bekannten Lösungs-, Misch-, Granulier-oder Dragierverfahren hergestellt.

Für eine orale Anwendungsform werden die aktiven Verbindungen mit den dafür üblichen Zusatzstoffen wie Trägerstoffen, Stabilisatoren oder inerten Verdünnungsmitteln vermischt und durch übliche Methoden in geeignete Darreichungsformen gebracht, wie Tabletten, Dragees, Steckkapseln, wäßrige, alkoholische oder ölige Suspensionen oder wäßrige, alkoholische oder ölige Lösungen. Als inerte Träger können z.B. Gummi arabicum, Magnesiumcarbonat, Kaliumphosphat, Milchzucker, Glucose, Magnesiumstearylfumarat oder Stärke, insbesondere Maisstärke verwendet werden. Dabei kann die Zubereitung sowohl als Trocken-oder Feuchtgranulat erfolgen. Als ölige Trägerstoffe oder Lösungsmittel kommen beispielsweise pflanzliche oder tierische Öle in Betracht, wie Sonnenblumenöl und Lebertran.

Zur subkutanen oder intravenösen Applikation werden die aktiven Verbindungen oder deren physiologisch verträgliche Salze, gewünschtenfalls mit den dafür üblichen Substanzen wie Lösungsvermittler, Emulgatoren oder weitere Hilfsstoffe in Lösung, Suspensionen oder Emulsionen gebracht. Als Lösungsmittel kommen z.B. in Frage: Wasser, physiologische Kochsalzlösungen oder Alkohole, z.B. Ethanol, Propandiol oder Glycerin, daneben auch Zuckerlösungen wie Glucose-oder Mannitlösungen, oder auch eine Mischung aus den verschiedenen genannten Lösungsmitteln.

<u>Verzeichnis der verwendeten Abkürzungen:</u>

AA Aminosäureanalyse
Ac Acetyl
ACHPA [3S,4S]-4-Amino-3-hydroxy-5-cyclohexyl-pentansäure
AHPPA [3S,4S]-4-Amino-3-hydroxy-5-phenyl-pentansäure
BOC tert.-Butoxycarbonyl
DC Dünnschichtchromatographie
DCC Dicyclohexylcarbodiimid

DNP 2,4-Dinitrophenyl
DMF Dimethylformamid
DMSO Dimethylsulfoxid
EE Essigsäureethylester
FAB Fast atom bombardment
Fmoc 9-Fluorenylmethyloxycarbonyl
HOBt 1-Hydroxybenzotriazol
NEM N-Ethylmorpholin
M Molekularpeak
MeOH Methanol
MS Massenspektrum
R.T. Raumtemperatur
Schmp. Schmelzpunkt
Sta [3S,4S]-4-Amino-3-hydroxy-6-methyl-heptansäure
Tic D,L-1,2,3,4-Tetrahydroisochinolin-3-carbonsäure
THF Tetrahydrofuran
Z Benzyloxycarbonyl

Die sonstigen für Aminosäuren verwendeten Abkürzungen entsprechen dem in der Peptidchemie üblichem drei Buchstaben-Code wie er z.B. in Europ. J. Biochem. 138, 9 -37 (1984) beschrieben ist. Falls nicht ausdrücklich anders angegeben handelt es sich immer um Aminosäuren der L-Konfiguration.

Die nachstehenden Beispiele dienen zur Erläuterung der vorliegenden Erfindung, ohne daß diese darauf beschränkt wäre.

## Beispiel 1:

(2S)-2-Benzyloxy-3-phenyl-propanoyl-His-Sta-Ile-2-pyridylmethylamid

160 mg (2S)-2-Benzyloxy-3-phenyl-propanoyl-His(DNP)-Sta-Ile-2-pyridylmethylamid werden in 7 ml DMF mit 0,6 ml Thiophenol 2 h bei R.T. gerührt. Man engt im Vakuum ein und digeriert 3 mal mit Diisopropylether. Nach Chromatographie an Kieselgel (Laufmittel EE/Methanol 6:1) und Einengen der produkthaltigen Fraktionen wird (2S)-2-Benzyloxy-3-phenylpropanoyl-His-Sta-Ile-2-pyridylmethylamid als farbloses Pulver erhalten.

$R_f$ (EE/Methanol 6 : 1) = 0,4 -0,5. MS (FAB) : 754 (M + 1).

Das Ausgangsmaterial (2S)-2-Benzyloxy-3-phenyl-propanoyl-His(DNP)-Sta-Ile-2-pyridylmethylamidwird nach der folgenden Arbeitsvorschrift hergestellt:

a) 85 mg (2S)-2-Benzyloxy-3-phenyl-propansäure, 226 mg H-His(DNP)-Sta-Ile-2-pyridylmethylamid und 83 mg HOBT werden in 15 ml THF gelöst. Bei 0 bis -5°C werden 91 mg DCC, gelöst in 10 ml THF, zugetropft. Der Ansatz steht über Nacht bei R.T. Man filtriert, engt im Vakuum ein, nimmt mit EE auf, extrahiert 2 mal mit 2n wäßr. Kaliumcarbonatlösung, 1 mal mit Wasser, trocknet über Magnesiumsulfat und engt ein. Nach Chromatographie an Kieselgel erhält man (2S)-2-Benzyloxy-3-phenyl-propanoyl-His(DNP)-Sta-Ile-2-pyridylmethylamid als schwach gelbliches Pulver. MS (FAB) : 920 (M + 1).

b) Herstellung von H-His(DNP)-Sta-Ile-2-pyridylmethylamid:

340 mg BOC-His-(DNP)-Sta-Ile-2-pyridylmethylamid werden in 10 ml Dioxan gelöst. Unter Eiskühlung werden 10 ml mit HCl gestättigtes Dioxan zugetropft. Nach 2 h bei R.T. wird im Vakuum eingeengt, in Wasser gelöst, einmal mit $CH_2Cl_2$ extrahiert, mit ges. wäßr. $Na_2CO_3$-Lösung auf pH 9 gestellt und 3 mal mit $CH_2Cl_2$ extrahiert. Nach Trocknung über $MgSO_4$ und Einengen im Vakuum erhält man H-His(DNP)-Sta-Ile-2-pyridylmethylamid als gelben Schaum, der ohne weitere Reinigung in den nächsten Kupplungsschritten weiter eingesetzt werden kann. MS (FAB) : 682 (M + 1).

c) Herstellung von BOC-His(DNP)Ile-2-pyridylmethylamid:

Zu 6,1 g BOC-His(DNP)-OH, 5,5 g H-Sta-Ile-2-pyridylmethylamid und 3,3 g HOBT werden in 100 ml THF bei 0 -5°C 3,7 g DCC, gelöst in 70 ml THF, zugetropft. Nach 4 h bei R.T. wird filtriert, eingeengt und in EE gelöst. Man extrahiert 2 mal mit 2 n wäßr. $K_2CO_3$-Lösung, einmal mit Wasser, trocknet über $MgSO_4$ und engt im Vakuum ein. Nach Chromatographie an Kieselgel (Laufmittel EE) werden 8 g (70 % Ausbeute) Boc-His(DNP)-Sta-Ile-2-pyridylmethylamid erhalten. $R_f$(EE/Methanol 10 : 1) = 0,37 MS (FAB) : 782 (M + 1).

d) Herstellung von H-Sta-Ile-2-pyridylmethylamid:

Analog b) ausgehend von 1 g BOC-Sta-Ile-2-pyridylmethylamid wird H-Sta-Ile-2-pyridylmethylamid als farbloser Feststoff erhalten. Schmp.: 132°C.

e) Herstellung von BOC-StaIle-2-pyridylmethylamid:

Aus 7,7 g BOC-Sta-OH [Herstellung nach J. Org. Chem. 43, 3624 (1978)], 6,2 g H-Ile-2-pyridylmethylamid, 6,2 g HOBT und 6,9 g DCC werden analog Vorschrift (c) 12,1 g BOC-Sta-Ile-2-pyridylmethylamid erhalten. Schmp.: 173°C.

f) Herstellung von H-Ile-2-pyridylmethylamid

Aus 6,6 g BOC-Ile-2-pyridylmethylamid wird analog Vorschrift (b) H-Ile-2-pyridylmethylamid erhalten. $R_f$-(EE/Methanol 1 : 1) = 0,3 -0,4.

g) Herstellung von BOC-Ile-2-pyridylmethylamid

Aus 20 g BOC-Ile-OH, 9,35 g 2-Picolylamin, 16,5 g HOBT und 19,9 g DCC werden analog Vorschrift (c) 18,5 g (67 %) Boc-Ile-2-pyridylmethylamid erhalten. MS : 321 (M).

Die Verbindungen der Beispiele 2 -19 werden analog Beispiel 1 ausgehend von H-His-(DNP)-Sta-Ile-2-pyridylmethylamid und dem entsprechenden N-terminalen Aminosäure bzw. Carbonsäurederivate hergestellt.

Beispiel Nr. Verbindung

2 2-Benzyl-6,6-dimethyl-4-oxo-heptanoyl-His-Sta-Ile-2-pyridylmethylamid
3 BOC-(Me)Phe-His-Sta-Ile-2-pyridylmethylamid
4 BOC-D,L-β-2-Thienylalanin-His-Sta-Ile-2-pyridylmethylamid
5 N-3-Phenylpropyl-Phe-His-Sta-Ile-2-pyridylmethylamid
6 N-2,3-Dichlor-4-(4-chloro-3-methylsulfonylbenzoyl)-phenoxyacetyl-His-Sta-Ile-2-pyridylmethylamid
7 N-4-(2,3-Dichlor-phenoxy)-butanoyl-His-Sta-Ile-2-pyridylmethylamid
8 BOC-Tyr(Me)-His-Sta-Ile-2-pyridylmethylamid
9 BOC-Tic-His-Sta-Ile-2-pyridylmethylamid
10 BOC-Phg-His-Sta-Ile-2-pyridylmethylamid
11 BOC-Tyr(Bzl)-His-Sta-Ile-2-pyridylmethylamid
12 BOC-Tyr(t-But)-His-Sta-Ile-2-pyridylmethylamid
13 BOC-Phe(4-$NO_2$)-His-Sta-Ile-2-pyridylmethylamid
14 BOC-D-Trp-His-Sta-Ile-2-pyridylmethylamid
15 Benzyloxycarbonyl-(E)-dehydrophenylalkenyl-His-Sta-Ile-2-pyridylmethylamid
16 Benzyloxycarbonyl-(Z)-dehydrophenylalanyl-His-Sta-Ile-2-pyridylmethylamid
17 D,L-2-tert.-Butoxycarbonylamino-4-(2-thienyl)-butanoyl-His-Sta-Ile-2-pyridylmethylamid
18 BOC-D,L-β-3-thienylalanyl-His-Sta-Ile-2-pyridylmethylamid
19 Fmoc-Phe-His-Sta-Ile-2-pyridylmethylamid

Um die Strukturen der hergestellten Peptide zu bestätigen, wurden verschiedene analytische und spektroskopische Methoden angewendet. Die Ergebnisse sind in Tabelle 1 zusammengefaßt.

Tabelle 1:

| Bsp. Nr. | MS (FAB) | DC ($R_f$) | 1H-NMR a) | Sonst. |
|---|---|---|---|---|
| 2 | 760 (M+1) | 0,15 (EE/MeOH= 10:1) | + | |
| 3 | 777 (M+1) | 0,12 (EE/MeOH= 10:1) | + | |
| 4 | 769 (M+1) | 0,14 (EE/MeOH= 10:1) | + | |
| 5 | 781 (M+1) | 0,45 (EE/MeOH= 10:1) | + | |
| 6 | 936 (M+1) | 0,22 ($CH_2Cl_2$/MeOH/ AcOH/$H_2O$=85:15:1:1 | + | |
| 7 | 719 (M+1) | 0,28 ($CH_2Cl_2$(MeOH/ AcOH/$H_2O$=85:15:1:1 | + | Schmp.: 229-230°C |
| 8 | 793 (M+1) | 0,5 (Butanol/AcOH /$H_2O$=3:1:1) | + | |
| 9 | 775 (M+1) | 0,44 (Butanol/AcOH /$H_2O$=3:1:1) | + | |
| 10 | 749 (M+1) | 0,45 (Butanol/AcOH /$H_2O$=3:1:1) | + | |
| 11 | 869 (M+1) | 0,56 (Butanol/AcOH /$H_2O$=3:1:1) | + | AA;His=1, Tyr(Bzl)=0,82 |

| | | | | |
|---|---|---|---|---|
| 12 | 835 (M+1) | 0,45 (Butanol/AcOH /$H_2O$=3:1:1) | + | AA;His=1, Tyr=0,88 |
| 13 | 808 (M+1) | 0,49 (Butanol/AcOH /$H_2O$=3:1:1) | + | C,H,N-Analyse +/- 0,3 |
| 14 | 802 (M+1) | 0,48 (Butanol/AcOH /$H_2O$=3:1:1) | + | AA;His=1 Trp:0,86 |
| 15 | 795 (M+1) | 0,14 (EE/MeOH=10:1) | + | |
| 16 | 795 (M+1) | 0,14 (EE/MeOH=10:1) | + | |
| 17 | 783 (M+1) | 0,15 (EE/MeOH=10:1) | + | |
| 18 | 769 (M+1) | 0,14 (EE/MeOH=10:1) | + | |
| 19 | 854 (M+1) | 0,3 (EE/MeOH=10:1) | + | |

a) gemessen bei 270 MHz; + bedeutet: in Einklang mit der angegebenen Struktur

Beispiel 20

BOC-Phe-β-2-thienylalanyl-Sta-Ile-2-pyridylmethylamid

0,7 g BOC-Phe-β-2-thienylalanin, 0,68 g H-Sta-Ile-2-pyridylmethylamid (Beispiel 1d), 160 mg HOBT und 176 mg DCC werden analog Beispiel 1c miteinander umgesetzt. Nach Chromatographie an Kieselgel - (Laufmittel Toluol/EE 1 : 1) erhält man die Titelverbindung als farblosen Feststoff. Schmp. 205°C. Das als Ausgangsverbindung dienende BOC-Phe-β-2-thienylalanin-OH wird nach der Aktivestermethode aus BOC-Phe-ONSucc und L-β-2-Thienylalanin hergestellt (J. Amer. Chem. Soc. 96, 1839).

Beispiele 21 -23

Analog Beispiel 20 werden hergestellt:

Beispiel Nr.

21 BOC-Phe-Tyr(Me)-Sta-Ile-2-pyridylmethylamid
22 BOC-Phe(4-Cl)-Sta-Ile-2-pyridylmethylamid
23 BOC-Phe-D,L-3-thienylalanin-Sta-Ile-2-pyridylmethylamid

Die analytischen Daten der Verbindungen der Beispiele 20 -23 sind in Tabelle 2 zusammengefaßt.

Beispiel 24

N-Acetyl-(2S,3S,6S)-octahydrocyclopenta[b]pyrrol-2-carbonyl-Phe-β-2-thienylalanyl-Sta-Ile-2-pyridylmethylamid

Aus $H_2N$-Phe-β-2-thienylalanyl-Sta-Ile-2-pyridylmethylamid (Herstellung aus Beispiel 20 analog Arbeitsvorschrift 1b) und N-Acetyl-(2S,3aS,6aS)-octahydrocyclopenta[b]pyrrol-2-carbonsäure (hergestellt nach EP-A-84164) wird die Titelverbindung durch DCC/HOBT-Kupplung analog der Vorschrift 1a als farbloser Feststoff erhalten. MS (FAB) : 838 (M+1).

Beispiele 25 -32

Analog Beispiel 24 werden die Verbindungen der Beispiele 25 -32 hergestellt.

Beispiel Nr.

25 6-Aminohexanoyl-Phe-β-2-thienylalanyl-Sta-Ile-2-pyridylmethylamid
26 Dimethylaminoacetyl-Phe-β-2-thienylalanyl-Sta-Ile-2-pyridylmethylamid
27 Fmoc-Phe-β-2-thienylalanyl-Sta-Ile-2-pyridylmethylamid
28 Z-Phe-β-2-thienylalanyl-Sta-Ile-2-pyridylmethylamid
29 Cyclopentanoyl-Phe-β-2-thienylalanyl-Sta-Ile-2-pyridylmethylamid
30 (2S)-trans-4-Hydroxy-1-acetylpyrrolidinyl-2-carbonyl-Phe-β-2-thienylalanyl-Sta-Ile-2-pyridylmethylamid
31 2-(o,o-Dichloroanilino)-phenylacetyl-Phe-β-2-thienylalanyl-Sta-Ile-pyridylmethylamid
32 N-Acetyl-(2R,3aR,6aR)-octahydrocyclopentan[b]pyrrol-2-carbonyl-Phe-β-2-thienylalanyl-Sta-Ile-2-pyridylmethylamid

Die analytischen Daten der Verbindungen der Beispiele 25 - 32 sind in Tabelle 2 zusammengefaßt.

Tabelle 2:

| Bsp. Nr. | MS (FAB) | DC ($R_f$) | 1H-NMR a) | Sonst. |
|---|---|---|---|---|
| 20 | 779 (M+1) | 0,28 (EE) | + | Schmp.:205°C |
| 21 | 803 (M+1) | 0,12 (EE) | + | |
| 22 | 807 (M+1) | 0,5 (EE) | + | |
| 23 | 779 (M+1) | 0,3 (EE) | + | |
| 24 | 858 (M+1) | 0,3 (EE/MeOH=10:1) | + | |
| 25 | 792 (M+1) | 0,2 (EE/MeOH=5:1) | + | |
| 26 | 764 (M+1) | 0,4 (EE/MeOH=10:1) | + | |
| 27 | 901 (M+1) | 0,41 (EE) | + | |
| 28 | 813 (M+1) | 0,37 (EE) | + | |
| 29 | 775 (M+1) | 0,28 (EE) | + | |
| 39 | 837 (M+1) | 0,4 (EE/MeOH=10:1) | + | |
| 31 | 955 (M+1) | 0,3 (Toluol/EE=3:1) | + | |
| 32 | 858 (M+1) | 0,35 (EE/MeOH=10:1) | + | |

a) gemessen bei 270 MHz; + bedeutet: in Einklang mit der angegebenen Struktur

Beispiel 33

BOC-Phe-His-Sta-[(3S,4S)-4-methyl-1-phenyl-(E)-1-hexen-3-yl]-amid

Aus 300 mg BOC-Phe-His(DNP)-Sta-[(3S,4S)-4-methyl-1-phenyl-(E)-1-hexen-3-yl]-amid erhält man durch DNP-Abspaltung analog Vorschrift 1 die Titelverbindung nach Chromatographie an Kieselgel - (Laufmittel EE/MeOH = 20 : 1 ) als farblosen Feststoff. M(FAB) : 731 (M+1). $R_f$ = 0,2 -0,3 (EE/MeOH = 20 : 1).

BOC-Phe-His(DNP)-Sta-[(3S,4S)-4-methyl-1-phenyl-(E)-1-hexen-3-yl]-amid wird durch DCC/HOBT-Kopplung aus BOC-PHE-His(DNP)-OH und des komplementären Statinamids hergestellt, dessen Synthese durch BOC-Abspaltung analog Vorschrift 1b aus BOC-Sta-[(3S,4S)-4-methyl-1-phenyl-(E)-1-hexen-3-yl]-amid erfolgt. Die Herstellung des vorstehenden Amides wird im folgenden beschrieben:

a) Zu 0,55 g BOC-Sta-OH, 0,45 g (3S,4S)-3-Amino-4-methyl-1-phenyl-(E)-1-hexen und 3,7 ml Triethylamin in 20 ml THF werden unter Eiskühlung 1,3 ml einer 50 prozentigen Lösung von Propanphosphonsäureanhydrid in Methylenchlorid zugetropft. Man läßt über Nacht bei R.T. stehen, engt im Vakuum ein und nimmt mit EE auf. Die organische Phase wird 3 mal mit ges. wäß. $Na_2CO_3$-Lösung und einmal mit Wasser extrahiert. Man trocknet über $MgSO_4$, engt im Vakuum ein und chromatographiert an Kieselgel - (Laufmittel Methylenchlorid/EE = 8 : 1). Durch Einengen der produkt haltigen Fraktionen wird BOC-Sta-[-(3S,4S)-4-methyl-1-phenyl-(E)-1-hexen-3-yl]-amid als farbloser Schaum erhalten. MS(FAB) : 447 (M+1). $R_f$ = 0,16 (Methylenchlorid/EE = 8 : 1).

b) <u>Herstellung von (3S,4S)-3-Amino-4-methyl-1-phenyl-(E)-1-hexen</u>:
Aus 0,5 g (3S,4S)-3-N-tert.-Butoxycarbonylamino-4-methyl-1-phenyl-(E)-1-hexen durch Abspaltung der BOC-Schutzgruppe analog Vorschrift 1b.

| | | | | | |
|---|---|---|---|---|---|
| $C_{13}H_{19}N*HCl$ (225,75) | Ber. | C69,16 | H8,48 | N6,21 | HCl16,15 |
| | Gef. | 69,1 | 8,4 | 6,0 | 16,4 |

c) <u>Herstellung von (3S,4S)-3-N-tert.-Butoxycarbonylamino-4-methyl-1-phenyl-(E)-1-hexen</u>
5,8 g Benzyltriphenylphosphoniumbromid und 1,37 g Kalium-tert.-butylat werden 2 h in abs. THF unter Argon bei R.T. gerührt. Bei R.T. wird eine Lösung von 2,18 g BOC-Isoleucinal (hergestellt nach Synthesis <u>1983</u>, 676) in 17 ml abs. THF zugetropft. Nach 30 min. bei R.T. wird mit 5 %iger wäßr. $NaHSO_4$-Lösung angesäuert und 3 mal mit Methylenchlorid extrahiert. Die organische Phase wird 1 mal mit 0,1 n HCl und 2 mal mit ges. $NaHCO_3$-Lösung extrahiert, über $MgSO_4$ getrocknet und im Vakuum eingeengt. Nach Chromatographie an Kieselgel erhält man die Titelverbindung als farblosen Feststoff.

| | | | | |
|---|---|---|---|---|
| $C_{18}H_{27}NO_3$ (289,42) | Ber. | C74,70 | H9,40 | N4,83 |
| | Gef. | 74,2 | 9,4 | 4,9 |

<u>Beispiel 34</u>

<u>BOC-Phe-His-Sta-[(3S,4S)-4-methyl-1-phenyl-hexan-3-yl]-amid</u>

Aus 80 mg Beispiel 33 erhält man die Titelverbindung durch Hydrierung bei Normaldruck in Methanol mit Pd/C als farblosen Feststoff.MS(FAB) : 733 (M+1). $R_f$ = 0,43 (EE/MeOH = 5 : 1).

<u>Beispiel 35</u>

BOC-Phe-His-Sta-[(3S)-1-phenyl-(E)-1-buten-3-yl]-amid

Ausgehend von BOC-Alaninal erhält man die Titelverbindung analog der in Beispiel 33 beschriebenen Reaktionssequenz. MS(FAB) : 689 (M+1). $R_f$ = 0,2 (EE/MeOH= 10 : 1).

Beipiele 36 -50

Analog den Bespielen 33 -35 werden die folgenden Verbindungen hergestellt:

Beispiel Nr.

36 BOC-Phe-His-Sta-3-phenyl-propylamid
37 BOC-Phe-His-Sta-[(3S,4S)-3-methyl-8-phenyl-octan-4-yl]amid
38 BOC-Phe-His-Sta-[(3S,4S)-3-methyl-7-phenyl-heptan-4-yl]-amid
39 BOC-Phe-His-Sta-(1S)-1-methyl-3-phenyl-propylamid
40 BOC-Phe-His-Sta-(1S)-1-methyl-3-(4-methoxy-phenyl)-propylamid
41 BOC-Phe-His-ACHPA-(1S)-1-methyl-3-phenyl-propylamid
42 BOC-Phe-His-ACHPA-(1S)-1-methyl-3-phenyl-propylamid
43 BOC-Phe-β-2-thienylalanyl-ACHPA-(1S)-1-methyl-3-phenylpropylamid
44 2-Benzyl-3-phenylpropanoyl-His-ACHPA-(1S)-1-methyl-3-phenyl-propylamid
45 BOC-Phe-His-Sta-[(3S,4S)-3-methyl-8-phenyl-(Z)-5-octen-4-yl]-amid
46 BOC-Phe-β-2-thienylalanin-ACHPA-(1S)-1-methyl-3-(2-pyridyl)-propylamid
47 BOC-Phe-β-2-thienylalanyl-ACHPA-[(3S,4S)-3-methyl-6-phenyl-4-hexyl]-amid
48 Z-Phe-His-ACHPA-(1S)-1-methyl-3-(2-pyridyl)-propylamid
49 BOC-Phe-His-Sta-(1R,S)-1-methyl-2-phenoxy-ethylamid
50 BOC-Phe-His-Sta-N-methyl-[(2S)-2-methyl-1-(4-methoxyphenyl)-ethan-2-yl]-amid

Die analytischen Daten der Verbindungen der Beispiele 33 - 50 werden in Tabelle 3 zusammengefaßt.

Tabelle 3:

| Bsp. Nr. | MS (FAB) | DC ($R_f$) | 1H-NMR a) | Sonst. |
|---|---|---|---|---|
| 33 | 731 (M+1) | 0,2-0,3 (EE/MeOH= 20:1) | + | |
| 34 | 733 (M+1) | 0,43 (EE/MeOH=5:1) | + | |
| 35 | 689 (M+1) | 0,2 (EE/MeOH=10:1) | + | |
| 36 | 677 (M+1) | 0,18 (EE/MeOH=10:1) | + | |
| 37 | 760 (M+1) | 0,46 (EE/MeOH=10:1) | + | |
| 38 | 746 (M+1) | 0,4 (EE/MeOH=10:1) | + | |
| 39 | 691 (M+1) | 0,2 ($CH_2Cl_2$/MeOH/ ACOH/$H_2O$=100/10/1/1) | + | |
| 40 | 707 (M+1) | 0,16 ($CH_2Cl_2$)/MeOH/ AcOH/$H_2O$=90:10:1:1 | + | Schmp.:140-145°C (Zers.) |
| 41 | 731 (M+1) | 0,25 ($CH_2Cl_2$/MeOH/ AcOH/$H_2O$=100/10/1:1) | + | |
| 42 | 741 (M+1) | 0,2 (Toluol/EE=1:1) | + | |
| 43 | 747 (M+1) | 0,19 (Toluol/EE=1:1) | + | |
| 44 | 705 (M+1) | 0,13 ($CH_2Cl_2$/MeOH) AcOH/$H_2O$=90:10:1:1 | + | |
| 45 | 758 (M+1) | 0,21 (EE/MeOH:10:1) | + | |

| | | | | |
|---|---|---|---|---|
| 46 | 748 (M+1) | 0,2 ($CH_2Cl_2$/MeOH=10:1) | + | |
| 47 | 789 (M+1) | 0,2 ($CH_2Cl_2$/EE=3:1) | + | |
| 48 | 761 (M+1) | 0,2 (EE/MeOH=20:1) | + | |
| 49 | 693 (M+1) | 0,19 ($CH_2Cl_2$/MeOH/) AcOH/$H_2O$=100:10:1:1) | + | Schmp.: 56-61°C |
| 50 | 721 (M+1) | 0,2 ($CH_2Cl_2$/MeOH/AcOH /$H_2O$=90:10:1:1) | + | Schmp.: 110°C (Zers.) |

a) gemessen bei 270 MHz; + bedeutet: in Einklang mit der angegebenen Struktur

Beispiel 51

BOC-Phe-β-2-Thienylalanyl-ACHPA-[(3S)-3-methyl-1-(3-ethoxycarbonylmethoxy)phenyl-propan-3-yl]-amid:

Aus BOC-Phe-β-2-Thienylalanin und H-ACHPA-[(3S)-3-methyl-1-(3-ethoxycarbonylmethoxy)-phenyl-propan-3-yl]-amid erhält man durch DCC/HOBT-Kupplung analog Beispiel 1a die Titelverbindung als farblosen Feststoff. MS(FAB : 849 (M+1). $R_f$= 0,1 (Toluol/EE= 1:1).H-ACHPA-[(3S)-3-methyl-1-(3-ethoxycarbonyl-methoxy)-phenyl-propan-3-yl]-amid wird durch BOC-Abspaltung der entsprechenden Vorstufe hergestellt, die ihrerseits durch DCC/HOBT-Kupplung aus BOC-ACHPA-OH und (2S)-2-Amino-4-(3-ethoxycarbonylmethoxy)-phenyl-butan synthetisiert wird. Die Herstellung des BOC-geschützten, vorstehend genannten Amines wird im folgenden beschrieben.

(2S)-2-tert.-Butoxycarbonylamino-4-(3-ethoxycarbonylmethoxy)-phenyl-buten:

Zu 1,12 g Kalium-tert.-butylat in 10 ml abs. THF werden unter Argon 5,5 g 3-(Methoxycarbonylmethoxy)-benzyl-triphenylphosphoniumjodid gelöst in 30 ml abs. THF bei R.T. zugetropft. Nach ½ h bei R.T. werden 1,6 g BOC-Alaninal, gelöst in 15 ml THF, zugetropft. Man rührt 1 h bei R.T., versetzt mit 5 %iger wäßr. $NaHSO_4$-Lösung und extrahiert 3 mal mit $CH_2Cl_2$. Die organische Phase wird 1 mal mit 0,1 n wäßr. HCl und 2 mal mit ges. wäßr. $NaHCO_3$-Lösung extrahiert, über $MgSO_4$ getrocknet und im Vakuum eingeengt. Nach Chromatographie an Kieselgel erhält man die Titelverbindung als farblosen Feststoff. MS:335 (M+).

3-(Methoxycarbonylmethoxy)-benzyl-triphenylphosphoniumjodid:

10 g 3-Jodmethyl-phenoxyessigsäuremethylester und 8,2 g Triphenylphosphin werden in 60 ml Toluol 1 h unter Rückfluß gekocht. Dabei scheidet sich das Produkt als Öl ab. Die Lösung wird abdekantiert und der ölige Rückstand an Kieselgel chromatographiert (Laufmittel: EE/MeOH=10:1). Man erhält die Titelverbindung als hellgelben, amorphen Feststoff. $R_f$= 0,31 (EE/MeOH=10:1).

3-Jodmethyl-phenoxyessigsäuremethylester:

12 g 3-(O-Tosyl-hydroxymethyl)-phenoxyessigsäureethylester und 6 g Kaliumjodid werden in 100 ml Aceton bis zur Vollständigkeit der Reaktion (D.C.-Kontrolle) unter Rückfluß gekocht. Man filtriert, engt im Vakuum ein und chromatrographiert an Kieselgel (Laufmittel: Toluol/EE = 10:1). Die Titelverbindung wird als farbloses Öl erhalten.

3-(O-Tosyl-hydroxymethyl)-phenoxyessigsäureethylester:

Zu 2,4 g Natriumhydrid in 100 ml abs. DMF werden bei 0°C 21,2 g 3-Hydroxymethyl-phenoxyessigsäuremethylester gelöst in 20 ml DMF zugetropft. Nach ½ h bei 0°C tropft man eine Lösung von 19 g p-Toluolsulfonsäurechlorid in 20 ml THF zu, rührt ½ h bei 0°C und 10 h bei R.T. Nach Einengen im Vakuum und Chromatographie des erhaltenen Rückstandes an Kieselgel (Laufmittel:Toluol/EE = 6:1) wird die Titelverbindung erhalten. $R_f$ = 0,5 (Toluol/EE = 4:1)

3-Hydroxymethyl-phenoxyessigsäuremethylester:

25 g 3-Hydroxybenzylalkohol und 24 g Kalium-tert.-butylat werden in 200 ml Ethanol gelöst. Bei R.T. werden 37 g Brom essigsäureethylester, gelöst in 100 ml Ethanol zugetropft. Nach 12 h bei R.T. engt man im Vakuum ein, nimmt mit Essigsäureethylester auf und entfernt die noch vorhandene Ausgangsverbindung durch zweimaliges Ausschütteln mit wäßr. 2n NaOH. Die verbleibende organische Phase wird 1 mal mit wäßr. 2N HCl, 1 mal mit Wasser ausgeschüttelt, über $MgSO_4$ getrocknet und im Vakuum eingeengt. Es werden 20 g der Titelverbindung erhalten. $R_f$ = 0,38 (Toluol/EE = 2:1).

Beispiel 52

BOC-Phe-β-2-Thienylalanyl-ACHPA-[(3S)-3-methyl-1-(3-carboxymethoxy)-phenyl-propan-3-yl]-amid: Zu 100 mg Beispiel 51, gelöst in 10 ml Dioxan, gibt man 3 ml wäßr. 0,1n NaOH und rührt bei R.T. bis zur vollständigen Verseifung des Methylesters (D.C.-Kontrolle). Es wird mit 50 ml Wasser verdünnt und 2 mal mit Essigester extrahiert. Man säuert die wäßrige Phase mit wäßr. 2n Zitronensäure an, extrahiert 3 mal mit $CH_2Cl_2$, trocknet über $MgSO_4$ und engt im Vakuum ein. Dabei wird die Titelverbindung als amorpher Feststoff erhalten. MS(FAB) : 821 (M + 1). $R_f$ = 0,3 (EE/MeOH = 10:1).

Beispiel 53

BOC-Phe-β-2-Thienylalanyl-ACHPA-[(3S)-3-methyl-1-(3-(9-fluorenylmethyloxycarbonylamino-methyl))-phenyl-propan-3-yl]-amid:

Die Synthese erfolgt analog der für Beispiel 51 angegebenen Reaktionssequenz aus BOC-Phe-β-2-thienylalanin und H-ACHPA-[(3S)-3-methyl-1-(3-(9-fluorenylmethyloxycarbonylamino-methyl))-phenyl-propan-3-yl]-amid. MS(FAB): 985 (M + 1). $R_f$ = 0,3 (Toluol/EE = 1:1). Die Herstellung der C-terminalen Aminkomponente wird nachstehend beschrieben.

(2S)-2-tert.-Butoxycarbonylamino-4-[3-(9-fluorenylmethyloxycarbonylamino-methyl)]-phenyl-butan:

0,5 g (2S)-2-tert.-Butoxycarbonylamino-4-(3-aminomethyl)-phenylbutan werden in 30 ml Dioxan/$H_2O$ = 1:1 gelöst und bei R.T. mit 0,3 g $NaHCO_3$ versetzt. Man fügt 0,61 g Fmoc-ONSucc hinzu und rührt über Nacht bei R.T. Anschließend wird filtriert, das Dioxan abrotiert, mit wäßr. 1n HCl angesäuert, mit $CH_2Cl_2$ extrahiert und über $MgSO_4$ getrocknet. Nach Chromatographie an Kieselgel wird das Produkt als farbloser Feststoff erhalten. MS(FAB) : 501(M + 1). $R_f$ = 0,4 (Toluol/EE = 8:1).

(2S)-2-tert.-Butoxycarbonylamino-4-(3-aminomethyl)-phenyl-butan:

1 g (2S)-2-tert.-Butoxycarbonylamino-4-(3-cyano)-phenyl-butan wird in 50 ml Ethanol mit Raney-Nickel bei 2 bar hydriert. Nach Filtration engt man im Vakuum ein, nimmt mit 100 ml EE auf und extrahiert 2 mal mit wäßr. 2n Zitronensäure. Es wird mit 2n NaOH alkalisch gestellt und 3 mal mit $CH_2Cl_2$ extrahiert. Trocknung mit $MgSO_4$ und Einengen im Vakuum ergibt das Produkt als gelbes Öl, welches ohne weitere Reinigung in den vorstehend beschriebenen Reaktionsschritt eingesetzt wird. MS : 278 (M+).

(2S)-2-tert.-Butoxycarbonylamino-4-(3-cyano)-phenyl-butan:

2,4 g (2S)-2-tert.-Butoxycarbonylamino-4-(3-cyano)-phenyl-(E/Z)-3-buten werden in 100 ml Methanol mit Pd/C bei R.T. unter Normaldruck hydriert. Man filtriert, engt im Vakuum ein, nimmt mit EE auf, extrahiert 2 mal mit wäßr. 2n Zitronensäurelösung, extrahiert 1 mal mit Wasser, trocknet über $MgSO_4$ und engt erneut im Vakuum ein. Als Rückstand verbleiben 2,3 g der Titelverbindung. MS : 274(M+). $R_f$ = 0,63 - (Toluol/EE=2:1).

(2S)-2-tert.-Butoxycarbonylamino-4-(3-cyano-phenyl)-(E/Z)-3-buten:

3,18 g Kalium-tert.-butylat und 13,3 g 3-Cyano-benzyl-triphenylphosphoniumbromid werden unter Argon in 160 ml abs. THF 1 h bei R.T. gerührt. Anschließend wird eine Lösung von 4,5 g Boc-Alaninal in 50 ml abs. THF bei R.T. zugetropft. Nach 1 h bei R.T. versetzt man das Re aktionsgemisch mit 50 ml einer 2m $NaHSO_4$-Lösung, destilliert das THF im Vakuum ab, verdünnt mit Wasser, extrahiert 3 mal mit $CH_2Cl_2$, trocknet über $MgSO_4$ und engt im Vakuum ein. Nach Chromatographie an Kieselgel (Laufmittel: EE=8:1) werden 4 g der Titelverbindung als E/Z-Isomerengemisch erhalten. MS : 272(M+).

3-Cyano-benzyl-triphenylphosphoniumbromid:

10 g 3-Brommethyl-acetonitril und 15 g Triphenylphosphin werden in 150 ml Toluol 1 h bei R.T. gerührt. Anschließend kocht man 2 h unter Rückfluß, kühlt ab, filtriert das ausgefallene Phosphoniumsalz und trocknet es im Vakuum. Es werden 20 g Produkt erhalten. Schmp.: 315 -317°C.

Beispiel 54

BOC-Phe-β-2-Thienylalanyl-ACHPA-[(3S)-3-methyl-1-(3-aminomethyl)-phenyl-propan-3-yl]-amid:

60 mg der Verbindung aus Beispiel 53 werden in 10 ml DMF unter Zusatz von 2 ml Morpholin bei R.T. gerührt. Man engt im Vakuum ein, digeriert mit Petrolether, dekantiert den Petrolether ab und engt erneut im Vakuum ein. Nach Chromatographie an Kieselgel (Laufmittel: EE/MeOH 5:1) erhält man die Titelverbindung als farblosen Feststoff. MS(FAB) : 721 (m+1). $R_f$=0,3 -0,4 (EE/MeOH= 4:1).

Analog dem vorstehend beschriebenen Schema wird die Verbindung des Beispiels 55 hergestellt:
2-Phe-His-ACHPA-[(3S)-3-methyl-1-(3-aminomethyl)-phenylpropan-3-yl]-amid. MS(FAB) : 793 (M+1). $R_f$ = 0,3 -0,4 (EE/MeOH= 5:1)

Beispiel 56

BOC-Phe-Tyr-AHPPA-Leu-2-pyridylmethylamid:

107,1 mg BOC-Phe-Tyr-OH werden in 3 ml DMF gelöst und 50,7 mg HOBt sowie 51,6 mg DCC zugegeben. Nach 1h Reaktionszeit werden 153,6 mg H-AHPPA-Leu-2-pyridylmethylamid-Hydrochlorid und 0,25 ml NEM eingebracht und die Lösung über Nacht gerührt. Anschließend wird vom ausgefallenen Harnstoff abfiltriert, das Filtrat eingeengt und der Rückstand in Essigsäureethylester aufgenommen. Die

organische Phase wird mit wäßr. Bicarbonatlösung und gesättigter wäßr. Natriumchloridlösung ausgeschüttelt, über Magnesiumsulfat getrocknet und eingeengt. Säulenchromatographische Reinigung an Kieselgel mit Methylenchlorid (100/5) als Laufmittel ergibt das gewünschte Produkt als farbloses Pulver. $R_f$=0,65 im Laufmittel Methylenchlorid (90)/Methanol (12)/Wasser (0,8)/Essigsäure (0,8); MS(FAB) : 823 (M+1).

Die Synthese der für das obige Produkt verwendeten Ausgangsverbindungen und der weiter unten aufgeführten Beispiele erfolgte nach folgendem Schema.

a) H-AHPPA-Leu-2-pyridylmethylamid:

1,78 g BOC-AHPPA-Leu-2-pyridylmethylamid werden analog Beispiel 1b mit Dioxan/HCl zu H-AHPPA-Leu-2-pyridylmethylamid-Hydrochlorid umgesetzt. $R_f$= 0,27 im Laufmittel Methylenchlorid (80)/Methanol - (20)/(Essigsäure (1)/Wasser (1).

b) BOC-AHPPA-Leu-2-pyridylmethylamid:

1,63 g BOC-AHPPA-OH (hergestellt wie bei D. Rich und E.T.O. Sun in J. Med. Chem. 1980, 27 -33 beschrieben) werden mit 1,55 g H-Leu-2-pyridylmethylamid-Hydrochlorid analog Beispiel 47 umgesetzt. $R_f$ 0,49 im Laufmittel Methylenchlorid (90)/Methanol (10)/Essigsäure (1)

c) H-Leu-2-pyridylmethylamid-Hydrochlorid:

5,0 g BOC-Leu-2-pyridylmethylamid werden analog Beispiel 1b mit Dioxan/HCl versetzt. Man erhält das Produkt als schwachgelbes, hygroskopisches Pulver. $R_f$ = 0,30 im Laufmittel Methylenchlorid (70)/Methanol (30)/Essigsäure(1)/Wasser (1).

d) BOC-Leu-2-pyridylmethylamid:

8,0 g BOC-Leu-OH werden in 60 ml DMF gelöst und bei -15°C mit 3,57 ml Picolylamin, 23,5 ml NEM und 22,16 ml Propanphosphonsäureanhydrid versetzt. Man läßt bei Raumtemperatur über Nacht stehen, zieht das Lösungsmittel im Vakuum ab und nimmt den Rückstand in Essigsäureethylester auf. Die organische Phase wird mit wäßr. Bicarbonat-Lösung, $KHSO_4/K_2SO_4$-Lösung und gesättigter wäßr. NaCl-Lösung extrahiert, über Magnesiumsulfat getrocknet und eingeengt. Das verbleibende ölige Rohprodukt wird in Diisopropylether gelöst und durch vorsichtige Zugabe von n-Hexan zur Kristallisation gebracht. Man erhält rhombische Kristalle vom Schmp. 82 -83°C. $R_f$ = 0,41 im Laufmittel Essigsäureethylester (90)/Methanol - (10).MS(FAB) 321(M+H).

e) Boc-Phe-Tyr-OH

1,32 g BOC-Phe-OH wird analog Beispiel 47 mit 1,16 g Boc-Phe-Tyr-OMe Hydrochlorid zu BOC-Phe-OMe umgesetzt. Das nach Aufarbeitung erhaltene Rohprodukt wird direkt mit einer 50 %igen Lösung von wäßr. 1n NaOH in Dioxan zu BOC-Phe-Tyr-OH verseift. Zur Aufarbeitung wird der Ansatz auf 100 ml verdünnt und zweimal mit Ether ausgeschüttelt. Die wäßrige Phase wird mit wäßr. 2n Hcl auf pH 2,5 gestellt und mit Essigsäureethylester extrahiert. Nach Trocknen über $MgSO_4$, und Abziehen des Lösungsmittels erhält man das Produkt als farbloses Pulver. $R_f$ = 0,37 im Laufmittel Methylenchlorid (90)/Methanol (12)-/Essigsäure (0,8), Wasser (0,8); MS(FAB) 429(M+H).

f) N-(tert.-Butyloxycarbonyl)-[3S,4S]-4-amino-5-cyclohexyl-3-hydroxypentansäure (BOC-ACHPA-OH):

3,14 g BOC-ACHPA-OEt werden analog BOC-Sta-OEt mit wäßr. NaOH in Dioxan verseift. Nach Aufarbeitung wird das halbfeste Rohprodukt aus Essigsäureethylester/Diisopropylether umkristallisiert. Man erhält farblose feine nadelförmige Kristalle vom Schmp. 108 -110°C; Drehwert: [α]= -35,8° (c= 1, Methanol); $R_f$ 0,6 im Laufmittel Methylenchlorid (90)/Methanol (10)/Essigsäure (1); MS(FAB) 316(M+H).

N-(tert.-Butyloxycarbonyl)-[3S,4S]-4-amino-5-cyclohexyl-3-hydroxypentansäureethylester(BOC-ACHPA-OEt):

Die Herstellung erfolgt analog der Synthese von BOC-Sta-OEt aus BOC-Cyclohexylalaninal und dem Lithiumenolat von Essigsäureethylester. Dazu werden zu einer Lösung von 20 ml Diisopropylamin in 37 ml Tetrahydrofuran bei -50°C unter Schutzgas (Argon) 89,1 ml einer 1,6-molaren Lösung von n-Butyllithium in Hexan getropft. Nach 20 min wird auf -78°C abgekühlt und unter Einhaltung der Temperatur tropfenweise mit 13,9 ml Essigsäureethylester versetzt und noch 15 min nachgerührt. Anschließend wird bei -75°C eine Lösung von 17,2 g BOC-Cyclohexylalaninal in 60 ml Tetrahydrofuran zugetropft, 5 min nachgerührt und dann innerhalb 45 min mit 240 ml wäßr. HCl versetzt und bei 0°C auf pH 2.5 gestellt. Nach Erwärmen auf Raumtemperatur wird mit Ether extrahiert, die organische Phase mit gesättigter wäßr. Natriumchlorid-Lösung ausgeschüttelt und nach Trocknung über $MgSO_4$ eingeengt. Das Rohprodukt wird an Kieselgel - (Grace®, 31 µm) mit dem Laufmittelsystem Essigsäureethylester (1)/n-Hexan (9) in die Diastereomeren getrennt. Bei der Elution erhält man zuerst das gewünschte 3S,4S-Diastereomer als farbloses Öl. $R_f$=0,16 in Hexan (8)/Essigsäureethylester (2); Drehwert [α]: -32.7° (c= 1, Methanol).

Das später eluierte 3R,4S-Diastereomer erhält man als kristalline Substanz vom Schmp. 75 -77°C (Ether/Hexan) mit dem Drehwert [α]=-20.8° (c= 1, Methanol); $R_f$ = 0,12 in Hexan (8)/ Essigsäureethylester (2).

In Analogie zum obigen Beispiel wurden die nachfolgend aufgeführten Verbindungen durch Reaktion der entsprechenden Dipeptide oder acylierten Aminosäuren mit H-AHPPA-Leu-2-pyridylmethylamid bzw. H-ACHPA-Leu-2-pyridylmethylamid erhalten.

Beispiel Nr. Verbundung

57 BOC-Phe-Phe-AHPPA-Leu-2-pyridylmethylamid
58 BOC-Phe-cyclohexylglycyl-AHPPA-Leu-2-pyridylmethylamid
59 BOC-Phe-D,L-3-(2-pyridyl)alanyl-AHPPA-Leu-2-pyridylmethylamid
60 BOC-Phe-Trp-AHPPA-Leu-2-pyridylmethylamid
61 BOC-Phe-Leu-AHPPA-Leu-2-pyridylmethylamid
62 BOC-Phe-cyclohexylalanyl-AHPPA-Leu-2-pyridylmethylamid
63 BOC-Phe-Tyr(Me)-AHPPA-Leu-2-pyridylmethylamid
64 BOC-Phe-His(τ-Me)-AHPPA-Leu-2-pyridylmethylamid
65 BOC-Phe-Phg-AHPPA-Leu-2-pyridylmethylamid
66 BOC-Phe-Asn-AHPPA-Leu-2-pyridylmethylamid
67 BOC-Phe-D,L-3-(3-pyridyl)alanyl-AHPPA-Leu-2-pyridylmethylamid
68 BOC-Phe-Met-AHPPA-Leu-2-pyridylmethylamid
69 BOC-Phe-Met(O)-AHPPA-Leu-2-pyidylmethylamid
70 BOC-Phe-Met($O_2$)-AHPPA-Leu-2-pyridylmethylamid
71 BOC-Phe-Asp-ACHPA-Leu-2-pyridylmethylamid
72 BOC-Trp-His-AHPPA-Leu-2-pyridylmethylamid
73 BOC-D,L-3-(2-Naphthyl)alanyl-Ile-AHPPA-Leu-2-pyridylmethylamid
74 BOC-D,L-3-(1-Naphtyl)alanyl-Ile-AHPPA-Leu-2-pyridylmethylamid
75 3-Phenylpropionyl-Ile-AHPPA-Leu-2-pyridylmethylamid
76 Dibenzylacetyl-Ile-AHPPA-Leu-2-pyridylmethylamid
77 BOC-Phe-β-2-Thienylalanyl-AHPPA-Leu-2-pyridylmethylamid

Tabelle 4:

| Bsp. Nr. | MS (FAB) | DC ($R_f$) | 1H-NMR a) | Sonst. |
|---|---|---|---|---|
| 57 | 807 (M+1) | 0,20 ($CH_2Cl_2$:MeOH 10:1) | + | |
| 58 | 799 (M+1) | 0,38 (Isomer 1) 0,36 (Isomer 2) $CH_2Cl_2$:MeOH 100:8 | + | |
| 59 | 808 (M+1) | 0,20 (Isomer 1) 0,16 (Isomer 2) $CH_2Cl_2$:MeOH:AcOH 100:5:0,1 | + | |
| 60 | 846 (M+1) | 0,58 ($CH_2Cl_2$:MeOH 10:1) | + | |
| 61 | 773 (M+1) | 0,43 ($CH_2Cl_2$:MeOH 10:1) | + | |
| 62 | 813 (M+1) | 0,45 ($CH_2Cl_2$:MeOH 10:1) | + | Schmp. 128-131°C, Zers. |
| 63 | 837 (M+1) | 0,42 ($CH_2Cl_2$:MeOH 10:1) | + | Schmp. 185-188°C, Zers. |
| 64 | 811 (M+1) | 0,54 ($CH_2Cl_2$:MeOH) | + | |
| 65 | 793 (M+1) | 0,30 ($CH_2Cl_2$:MeOH 10:1) | + | |
| 66 | 774 (M+1) | 0,29 ($CH_2Cl_2$:MeOH: AcOH:$H_2O$ 100:10:1:1) | + | |
| 67 | 808 (M+1) | 0,44 (Isomer 1) 0,40 (Isomer 2) $CH_2Cl_2$:MeOH:AcOH 100:8:0,8) | + | |

| | | | | | |
|---|---|---|---|---|---|
| 68 | 791 (M+1) | 0,42 | ($CH_2Cl_2$:MeOH 10:1) | + | |
| 69 | 807 (M+1) | 0,40 | ($CH_2Cl_2$:MeOH 10:1) | + | Schmp. 183-188°C, Zers. |
| 70 | 823 (M+1) | 0,41 | ($CH_2Cl_2$:MeOH 10:1) | + | |
| 71 | 781 (M+1) | 0,19 | ($CH_2Cl_2$:MeOH: AcOH:$H_2O$ 100:10:1:1) | + | |
| 72 | 799 (M+1) | 0,31 | ($CH_2Cl_2$:MeOH: AcOH:$H_2O$ 100:15:1,5:1,5 | + | |
| 73 | 823 (M+1) | 0,38 | ($CH_2Cl_2$:MeOH 10:1) | + | |
| 74 | 823 (M+1) | 0,50 | ($CH_2Cl_2$:MeOH: AcOH:$H_2O$ 100:10:1:1) | + | |
| 75 | 658 (M+1) | 0,31 | ($CH_2Cl_2$:MeOH: AcOH 100:5:0,5) | + | Schmp. 187-190°C. |
| 76 | 748 (M+1) | 0,38 | ($CH_2Cl_2$:MeOH 9:1) | + | Schmp. 204-206°C |
| 77 | 812 (M+1) | 0,22 | (EE) | + | |

a) gemessen bei 60 bzw. 270 MHz; + bedeutet: in Einklang mit der angegebenen Stuktur.

Beispiel 78

Z-Phe-β-Thienylalanyl-Sta-Ile-8-tert.-butoxycarbonylaminooctylamid:

Aus Z-Phe-β-2-Thienylalanin und H-Sta-Ile-8-tert.-butoxycarbonylamino-octylamid wird die Titelverbindung analog Beispiel 56 durch DCC/HOBT-Kopplung als farbloser Feststoff erhalten. MS(FAB) : 951(M+1). $R_f$: 0,5 (EE).

Beispiel 79

Z-Phe-β-2-Thienylalanyl-Sta-Ile-8-amino-octylamid:

Aus 100 mg Beispiel 78 wird die Titelverbindung durch Abspaltung der Boc-Schutzgruppe analog Beispiel 1b als farbloses Pulver erhalten. MS(FAB) : 851(M+1). $R_f$: 0,2 (EE/MeOH = 5:1).

Beispiel 80

BOC-Phe-His-Sta-Leu-2-N-(2-pyrimidinyl)-amino-ethylamid:

Aus BOC-Phe-His(DNP)-OH und H-Sta-Leu-2-N-(2-pyrimidinyl)-aminoethylamid wird Boc-Phe-His(DNP)-Sta-Leu-2-N-(2-pyrimidinyl)-aminoethylamid analog Beispiel 56 durch DCC/HOBT-Kopplung als gelber Feststoff erhalten. Die Abspaltung der DNP-Schutzgruppe erfolgt wie in Beispiel 1 beschrieben. Die Titelverbindung wird als farbloser Feststoff nach säulenchromatographischer Reinigung erhalten. MS(FAB) : 793 - (M+1). $R_f$: 0.1 ($CH_2Cl_2$/MeOH/AcOH/$H_2O$ = 100:90:10:1). Schmp.: 126°C (Zers.).

Beispiel 81

N-Acetyl-(2S-3aS,6aS)-octahydrocyclopenta[b]pyrrol-2-carbonyl-Phe-Leu-Sta-(4-amido-1-benzyl-piperidin):

Aus N-Acetyl-(2S,3aS,6aS)-octahydrocyclopenta[b]pyrrol-2-carbonsäure und H-Phe-Leu-Sta-(4-amido-1-benzyl-piperidin) wird die Titelverbindung analog Beispiel 25 durch DCC/HOBT Kopplung als farbloser Feststoff erhalten. MS(FAB) : 900(M+1). $R_f$ = 0,4 ($CH_2Cl_2$/MeOH/AcOH/$H_2O$ = 85:1:1:1. [α] = -49.9° (c=1, MeOH).

Beispiel 82

BOC-Phe-His-Sta-Leu-Asn-$NH_2$:

Aus BOC-Phe-His(DNP)-OH und H-Sta-Leu-Asn-$NH_2$ wird BOC-Phe-His(DNP)-Sta-Leu-Asn-$NH_2$ analog Beispiel 56 durch DCC/HOBT-Kopplung als gelber Feststoff erhalten. Die Abspaltung der DNP-Schutzgruppe erfolgt wie in Beispiel 1 beschrieben. Die Titelverbindung wird als farbloser Feststoff nach säulenchromatographischer Reinigung isoliert. MS(FAB) : 786 (M+1). $R_f$ = 0,15-($CH_2Cl_2$/MeOH/AcOH/$H_2O$ = 80:20:1:1) Schmp.: 213 -215°C.

Analog Beispiel 82 werden die Verbindungen der Beispiele 83 -86 aus BOC-Phe-β-2-Thienylalanin und der korrespondierenden Aminokomponente hergestellt.

Beispiel Nr. Verbindung

83 BOC-Phe-β-2-Thienylalanyl-Sta-Ile-Arg-OH
84 BOC-Phe-β-2-Thienylalanyl-Sta-Ile-Arg-OMe
85 BOC-Phe-β-2-Thienylalanyl-Sta-Ile-Arginol
86 BOC-Phe-β-2-Thienylalanyl-Sta-Ile-Arg-$NH_2$

Die analytischen Daten der Verbindungen 83 -86 werden in Tabelle 5 zusammengefaßt.

Beispiel 87

Z-Phe-β-2-Thienylalanyl-Sta-Ile-3-(tert.-butoxycarbonylaminomethyl)-benzylamid:

Aus Z-Phe-β-2-Thienylalanin und H-Sta-Ile-3-(tert.-butoxycarbonylamino-methyl)-benzylamid wird die Titel verbindung durch DCC/HOBT-Kopplung analog Beispiel 20 nach chromatographischer Reinigung als farbloser Feststoff erhalten. MS(FAB) : 941(M+1). $R_f$= 0,3 (EE).

Beispiel 88

Z-Phe-β-2-Thienylalanyl-Sta-Ile-3-(aminomethyl)-benzylamid:

Aus 100 mg der Verbindung von Beispiel 87 wird die Titelverbindung durch BOC-Abspaltung analog Vorschrift 1b hergestellt. MS(FAB) : 841(M+1). $R_f$ :0,2(EE/MeOH=4:1)

Analog Beispiel 88 bzw. Beispiel 87 werden die Verbindungen der Beispiele 89 -91 synthetisiert. Ihre analytischen Daten werden in Tabelle 5 zusammengefaßt.

Beispiel Nr. Verbindung

89 Fmoc-Phe-β-2-Thienylalanyl-Sta-Ile-3-(aminomethyl)-benzylamid

90 Z-Phe-β-2-Thienylalanyl-ACHPA-Ile-3-(aminomethyl)-benzylamid

91 2-(9-Fluorenylmethyloxycarbonyl-amino)-4-(2-thienyl)-butanoyl-His-Sta-Ile-3-(aminomethyl)-benzylamid

Beispiel 92

Z-Phe-β-2-Thienylalanyl-ACHPAILe-3-(guanidinomethyl)-benzylamid:

Aus 100 mg der Verbindung von Beispiel 88 und 80 mg 1-Guanidyl-3,5-dimethyl-pyrazol-Salpetersäuresalz wird die Titelverbindung nach Literaturvorschrift erhalten (Houben-Weyl, Methoden der organischen Chemie, Band 15/1 S. 532). MS(FAB) : 883 (M+1).

Beispiel 93

Z-Phe-β-2-Thienylalanyl-StaIle-(3-tert.-butoxycarbonylmethoxy-)benzylamid:

345 mg Z-Sta-Ile-L-3-(tert.Butoxycarbonylmethoxy)-benzylamid werden durch katal. Hydrierung (MeOH, Pd/C) in $H_2^{N-}$ Sta-Ile-3-(tert.-butoxycarbonylmethoxy)-benzylamid überführt. Dieses wird ohne weitere Reinigung mit 315 mg Z-Phe-β-2-Thienylalanin-ONSucc zu der genannten Titelverbindung gekoppelt. MS(FAB) : 942(M+1). $R_f$= 0,3(EE).

Beispiel 94

Z-Phe-β-2-Thienylalanyl-Sta-Ile-3-(carboxy)-benzylamid:

100 mg der Verbindung von Beispiel 93 werden in 10 ml $CH_2Cl_2/CF_3CO_2H$= 1:1 bei R.T. bis zur vollständigen Reaktion gerührt (D.C.-Kontrolle). Nach Einengen im Vakuum wird die Titelverbindung als chromatographisch einheitlicher Feststoff isoliert. MS(FAB) : 886 (M+1). $R_f$= 0,4 (EE/MeOH= 4:1).

Analog Beispiel 93 bzw. Beispiel 94 werden die Verbindungen der Beispiele 95 -96 synthetisiert. Ihre analytischen Daten werden in Tabelle 5 zusammengefaßt.

Beispiel Nr. Verbindung

95 Z-Phe-β-2-Thienylalanyl-ACHPA-Ile-3-(tert.-butoxycarbonylmethoxy)-benzylamid
96 Z-Phe-β-2-Thienylalanyl-ACHPA-Ile-3-(Carboxymethoxybenzylamid

Tabelle 5

| Bsp. Nr. | MS (FAB) | DC ($R_f$) | 1H-NMR a) | Sonst. |
|---|---|---|---|---|
| 83 | 845 (M+1) | 0,2 ($CH_2Cl_2$:MeOH 10:1) | + | |
| 84 | 859 (M+1) | 0,5 ($CH_2Cl_2$:MeOH 10:1) | + | |
| 85 | 831 (M+1) | 0,4 ($CH_2Cl_2$:MeOH 10:1) | + | |
| 86 | 844 (M+1) | 0,2 ($CH_2Cl_2$:MeOH 8:1) | + | |
| 89 | 928 (M+1) | 0,4 (EE:MeOH=5:1) | + | |
| 90 | 869 (M+1) | 0,4 (EE:MeOH=20:1) | + | |
| 91 | 933 (M+1) | 0,4 (EE:MeOH=20:1) | + | |
| 95 | 965 (M+1) | 0,5 (EE:Toluol=10:1) | + | |
| 96 | 909 (M+1) | 0,3 ($CH_2Cl_2$:MeOH 10:1) | + | |

a) gemessen bei 60 bzw. 270 MHz; + bedeutet: in Einklang mit der angegebenen Struktur

Beispiel 97

Iva-Phe-His-ACHPA-3-(2-pyridyl)-propylamid

Aus 210 mg Iva-Phe-His(DNP)-ACHPA-3(2-pyridyl)-propylamid erhält man durch DNP-Abspaltung analog Vorschrift 1 die Titelverbindung nach Chromatographie an Kieselgel (Laufmittel $CH_3CN/H_2O = 8:1$) als farblosen Feststoff. $R_f$ ($CH_3CN/H_2O = 8:1$) = 0,2 . MS(FAB):702(M + 1).

Das Ausgangsmaterial Iva-Phe-His(DNP)-ACHPA-3-(2-pyridyl)-propylamid wird nach folgender Arbeitsvorschrift hergestellt:

a) Durch DCC/HOBt-Kupplung von Iva-Phe-His(DNP)-OH und H-ACHPA-3-(2-pyridyl)-propylamid erhält man analog Vorschrift 1a Iva-Phe-His(DNP)-ACHPA-3-(2-pyridyl)-propylamid nach Chromatographie an Kieselgel (Laufmittel EE/MeOH = 10:1) als gelben Feststoff.
$R_f$ (EE/MeOH = 10:1) = 0,3 . MS(FAB):868 (M + 1).

b) Herstellung von H-ACHPA-3-(2-pyridyl)-propylamid
Aus Boc-ACHPA-3-(2-pyridyl)-propylamid erhält man durch Abspaltung der BOC-Schutzgruppe analog Vorschrift 1b die Titelverbindung.

c) Herstellung von Boc-ACHPA-3-(2-pyridyl)-propylamid
Aus BOC-ACHPA-OH und 3-(2-pyridyl)-propylamin wird die Titelverbindung durch DCC/HOBt-Kupplung analog Beispiel 1c nach Chromatographie an Kieselgel (Laufmittel EE/MeOH = 10:1) als farbloser Feststoff erhalten. $R_f$ (EE/MeOH = 10:1) = 0,4 . MS(FAB):434(M + 1).

d) Herstellung von 3-(2-Pyridyl)-propylamin
Durch katalytische Hydrierung von Z-[3-(2-pyridyl)] -prop-2-enylamin analog Beispiel 34 erhält man die Titelverbindung, die ohne weitere Reinigung in den vorstehend beschriebenen Reaktionsschritt eingesetzt wird.

e) Herstellung von Z-[3-(2-pyridyl)] -prop-2-enylamin
532 mg Kalium-tert.-butylat und 1,95g 2-Picolyltriphenylphosphoniumchlorid werden 3h in 50ml THF unter Argon bei. R.T. gerührt. Bei 0°C wird eine Lösung von 870 mg Z-aminoacetaldehyd in 10 ml abs. THF zugetropft. Anschließend wird ohne Kühlung 90min gerührt, in Wasser gegeben und 3 mal mit $CH_2Cl_2$ extrahiert. Die organischen Extrakte werden nach dem Trocknen mit $Na_2SO_4$ und Einengen an Kieselgel chromatographiert (Laufmittel Toluol/EE = 1:1).
$R_f$ (Toluol/EE = 1:1) = 0,3 . MS:268($M^+$).

f) Herstellung von Z-aminoacetaldehyd
1g Z-aminoacetaldehyddimethylacetal werden in 20 ml 1n HCl und 5 ml THF 16h bei R.T. gerührt. Nach dreimaliger Extraktion mit EE werden die organischen Extrakte getrocknet ($Na_2SO_4$) und eingeengt. Die so erhaltene Titelverbindung wird ohne weitere Reinigung für den folgenden Reaktionsschritt verwendet.

Beipiele 98-101

Analog den Beispielen 33-35 werden die folgenden Verbindungen hergestellt. Ihre analytischen Daten werden in Tabelle 6 zusammengefaßt.

Beispiel Nr.

98 Ac-Phe-His-ACHPA-(3S)-[1-(2-pyridyl)-4-methylpentan-3-yl] -amid
99 Z-Phe-His-ACHPA-(3S)-[ 1-(2-pyridyl)-4-methyl-pentan-3-yl] amid
100 Iva-Phe-His-ACHPA-(3S)-[ 1-(2-pyridyl)]-butylamid
101 Boc-Phe-His-ACHPA-(3S)-[ 1-(2-pyridyl)]-butylamid

Tabelle 6

| Bsp.Nr. | MS(FAB) | DC($R_f$) | 1H-NMR a) | Sonst. |
|---|---|---|---|---|
| 98 | 702(M+1) | 0,1(EE:MeOH =5:1) | + | |
| 99 | 794(M+1) | 0,1(EE:MeOH =5:1) | + | |
| 100 | 716(M+1) | 0,1(EE:MeOH =4:1) | + | |
| 101 | 732(M+1) | 0,2(EE:MeOH =5:1) | + | |

a) + bedeutet: in Einklang mit der angegebenen Struktur

**Ansprüche**

1. Verbindung der Formel I

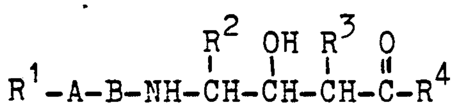

(I)

in welcher
$R^4$ $a_1$) einen Rest der Formel II oder III bedeutet,

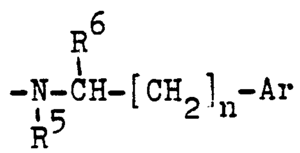

(II)

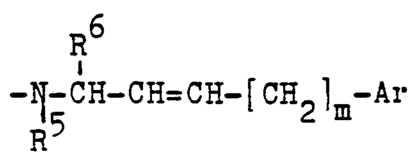

(III)

worin $R^5$ für Wasserstoff oder ($C_1$-$C_7$)-Alkyl und $R^6$ für Wasserstoff, ($C_1$-$C_7$)-Alkyl oder durch Hydroxy, ($C_1$-$C_5$)-Alkoxy, ($C_1$-$C_5$)-Alkylthio, Carboxy, ($C_1$-$C_5$)-Alkoxycarbonyl, Cl, Br, ($C_1$-$C_5$)-Alkylamino, Di-($C_1$-$C_5$)-alkylamino, ($C_1$-$C_5$)-Alkoxycarbonylamino oder ($C_1$-$C_{15}$)-Aralkyloxycarbonylamino monosubstituiertes ($C_1$-$C_7$)-Alkyl stehen,
m = 0, 1, 2, 3 oder 4 ist,
n = 1, 2, 3, 4, 5, oder 6 ist,
die Doppelbindung im Rest der Formel III im Falle n = 0 vorzugsweise die E-Konfiguration, andernfalls vorzugsweise die Z-Konfiguration aufweist und
Ar für ($C_6$-$C_{14}$)-Aryl, das gegebenenfalls durch einen oder zwei gleiche oder verschiedene Reste aus der Reihe F, Cl, Br, J, Hydroxy, ($C_1$-$C_7$)-Alkoxy, ($C_1$-$C_7$)-Alkyl, ($C_1$-$C_7$)-Alkoxycarbonyl, Amino, ($C_1$-$C_7$)-Alkylamino,

Di-($C_1$-$C_7$)-Alkylamino, Carboxy, Carboxymethyloxy, Amino-($C_1$-$C_7$)-alkyl, ($C_1$-$C_7$)-Alkylamino-($C_1$-$C_7$)-alkyl, Di-($C_1$-$C_7$)-alkylamino-($C_1$-$C_7$)-alkyl, ($C_1$-$C_7$)-Alkoxycarbonylmethoxy, Carbamoyl, Sulfamoyl, ($C_1$-$C_7$)-Alkoxysulfonyl, Sulfo und Guanidinomethyl substituiert ist, oder für den Rest eines 5-oder 6-gliedrigen monocyclischen oder 9-oder 10-gliedrigen bicyclischen Heteroaromaten mit mindestens 1 C-Atom, 1 -3 N-Atomen und/oder 1 S-oder O-Atom als Ringglieder steht, wobei Ar auch über -O-gebunden sein kann,

$a_2$) einen über eine Peptidbindung verknüpften Rest einer Aminosäure aus der Reihe Phenylalanin, Histidin, Tyrosin, Tryptophan, Methionin, Leucin, Isoleucin, Asparagin, Asparaginsäure, β-2-Thienylalanin, β-3-Thienylalanin, 4-Chlorphenylalanin, Methioninsulfon, Methioninsulfoxid, 2-Pyridylalanin, 3-Pyridylalanin, Cyclohexylalanin, Cyclohexylglycin, im-Methylhistidin, O-Methyltyrosin, O-Benzyltyrosin, O-tert.-Butyltyrosin, Phenylglycin, 1-Naphthylalanin, 2-Naphthylalanin, 4-Nitrophenylalanin, 2-Fluorphenylalanin, 3-Fluorphenylalanin, 4-Fluorphenylalanin, Norleucin, 1,2,3,4-Tetrahydroisochinolin-3-carbonsäure, Homophenylalanin, DOPA, O-Dimethyldopa, 2-Amino-4-(2-thienyl)-Buttersäure, 2-Amino-4-(3-thienyl)-buttersäure, 3-(2-Thienyl)-serin, (Z)-Dehydrophenylalanin und (E)-Dehydrophenylalanin, dessen C-terminale Carboxylgruppe frei, zur $CH_2OH$ reduziert oder

als Amid, welches einen C-Terminus der Formel $-NR^7R^8$ aufweist, vorliegt, worin $R^7$ und $R^8$ gleich oder verschieden sind und für

Wasserstoff,

($C_1$-$C_{10}$)-Alkyl, das gegebenenfalls durch Amino, ($C_1$-$C_5$)-Alkoxycarbonylamino, ($C_7$-$C_{15}$)-Aralkylcarbonylamino, Heteroarylamino, worin der Rest des Heteroaromaten wie oben unter ($a_1$) definiert ist, Hydroxy, ($C_1$-$C_5$)-Alkoxy, Sulfo, Carboxy oder ($C_1$-$C_5$)-Alkoxycarbonyl monosubstituiert ist,

($C_6$-$C_{14}$)-Aryl-($C_1$-$C_4$)-alkyl, das gegebenenfalls im Arylteil durch Amino, Carboxy, ($C_1$-$C_5$)-Alkoxycarbonyl, Amino-($C_1$-$C_5$)-alkyl, ($C_1$-$C_5$)-Alkoxycarbonylamino-($C_1$-$C_5$)-alkyl, ($C_7$-$C_{15}$)-Aralkoxycarbonylamino-($C_1$-$C_5$)-alkyl, ($C_1$-$C_5$)-Alkoxycarbonyl-($C_1$-$C_5$)-alkoxy, Carboxy-($C_1$-$C_5$)-alkoxy oder Guanido-($C_1$-$C_5$)-alkoxy monosubstituiert ist, oder

Heteroaryl-($C_1$-$C_4$)-alkyl, wobei der Rest des Heteroaromaten wie Ar unter ($a_1$) definiert ist, oder für einen Rest der Formel IV steht,

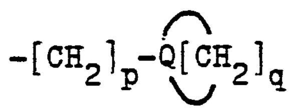 (IV)

worin p= 1, 2, 3 oder 4 ist, q= 4 oder 5 ist und Q N oder CH bedeutet oder p= 0 ist, 1 = 4 oder 5 ist und Q CH bedeutet und worin eine $CH_2$-Gruppe im Ring durch NH, O, S, CO, N-Bzl, N-Z oder N-BOC ersetzt sein kann, oder

$a_3$) einen über eine Peptidbindung verknüpften Rest eines Dipeptids aus der Reihe Leu-Asn, Ile-His, Ile-Arg und Ile-Lys bedeutet, dessen C-Terminus als freie COOH-Gruppe, die gegebenenfalls zur $CH_2OH$-Gruppe reduziert ist, als $CONH_2$ oder als $COOR^9$ mit $R^9$= ($C_1$-$C_7$)-Alkyl vorliegt und worin die ε-Aminofunktion des Lys gegebenenfalls durch ($C_1$-$C_5$)-Alkoxycarbonyl oder ($C_7$-$C_{15}$)-Aralkyloxycarbonyl geschützt ist;

$R^1$ $b_1$) abwesend ist oder Wasserstoff bedeutet,

$b_2$) ($C_1$-$C_{20}$)-Alkyl, das gegebenenfalls durch ein, zwei oder drei gleiche oder verschiedene Reste aus der Reihe Hydroxy, ($C_1$-$C_7$)-Alkoxy, ($C_1$-$C_7$)-Alkanoyloxy, Carboxy, ($C_1$-$C_7$)-Alkoxycarbonyl, ($C_1$-$C_8$)-Alkanoyloxy, Cl, Br, Amino, ($C_1$-$C_7$)-Alkylamino, Di-($C_1$-$C_7$)-alkylamino, ($C_1$-$C_5$)-Alkoxycarbonylamino oder ($C_7$-$C_{11}$)-Aralkyloxycarbonylamino substituiert ist,

($C_3$-$C_8$)-Cycloalkyl,

($C_3$-$C_8$)-Cycloalkyl-($C_1$-$C_{10}$)alkyl oder

($C_6$-$C_{14}$)-Aryl-($C_1$-$C_8$)-alkyl, das im Arylteil gegebenenfalls durch einen oder zwei gleiche oder verschiedene Reste aus der Reihe F, Cl, Br, Hydroxy, ($C_1$-$C_7$)-Alkoxy, ($C_1$-$C_7$)-Alkyl, ($C_1$-$C_7$)-Alkoxycarbonyl, Amino oder Trifluormethyl substituiert ist, bedeutet oder

$b_3$) einen Rest der Formel V bedeutet,

$R^a$ -W-(V)

worin W für -CO-, -O-CO-, $-SO_2$-oder -NH-CO-steht und $R^a$ Wasserstoff, ($C_1$-$C_{10}$)-Alkyl, das gegebenenfalls ein-oder zweifach ungesättigt ist und das gegebenenfalls durch bis zu 3 gleiche oder verschiedene Rest aus der Reihe Hydroxy, ($C_1$-$C_7$)-Alkoxy, ($C_1$-$C_7$)-Alkanoyloxy, Carboxy, ($C_1$-$C_7$)-Alkoxycarbonyl, Cl, Br, Amino, ($C_1$-$C_7$)-Alkylamino, Di-($C_1$-$C_7$)-Alkyl amino, ($C_1$-$C_5$)-Alkoxycarbonylamino, ($C_7$-$C_{15}$)-Aralkoxycarbonylamino oder 9-Fluorenylmethyloxycarbonylamino monosubstituiert ist,

($C_3$-$C_8$)-Cycloalkyl,

($C_3$-$C_8$)-Cycloalkyl-($C_1$-$C_6$)alkyl,

$(C_6-C_{14})$-Aryl, das gegebenenfalls durch einen oder zwei gleiche oder verschiedene Reste aus der Reihe F, Cl, Br, J, Hydroxy, $(C_1-C_7)$-Alkoxy, $(C_1-C_7)$-Alkyl, $(C_1-C_7)$-Alkoxycarbonyl, Amino, gegebenenfalls mit bis zu 2 Halogen substituiertes Anilino und Trifluormethyl substituiert ist,
$(C_6-C_{14})$-Aryl-$(C_1-C_6)$-alkyl, worin der Arylteil gegebenenfalls wie vorstehend bei Aryl definiert ist, Heteroaryl oder Heteroaryl-$(C_1-C_6)$-alkyl, wobei der Rest des Heteroaromaten jeweils wie Ar unter $(a_1)$ definiert ist, bedeutet, oder
$b_4$) einen Rest aus der Reihe (2S)-trans-4-Hydroxy-1-acyl-pyrrolidinyl-2-carbonyl, 5-Pyrrolidon-2S-carbonyl, α-Methoxycarbonyl-α-acylacetyl, 1-Acyl-(2R, 3aR, 6aR)-octahydro-cyclopenta[b]pyrrol-2-carbonyl und 1-Acyl-(2S,3aS,6aS)-octahydro-cyclo-penta[b]pyrrol-2-carbonyl bedeutet, wobei Acyl die Bedeutung $(C_1-C_6)$-Alkanoyl, $(C_1-C_5)$-Alkoxycarbonyl oder $(C_7-C_{15})$-Alkoxycarbonyl oder $(C_7-C_{15})$-Aralkyloxycarbonyl hat,
A $c_1$) einen Rest der Formeln VI oder VII bedeutet,

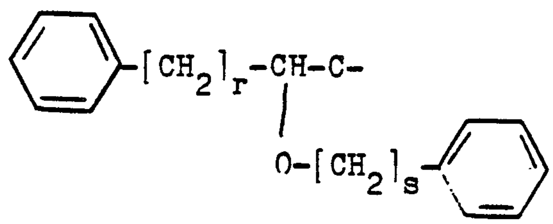
(VI)

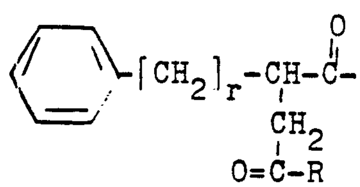
(VII)

in welchen
r = 1, 2 oder 3 ist,
s = 1, 2 oder 3 ist und
R $(C_1-C_7)$-Alkyl bedeutet, oder
$c_2$) einen N-terminal mit $R^1$ und C-terminal mit B verknüpften Rest einer Aminosäure bedeutet, die wie unter $(a_2)$ definiert ist,
B $d_1$) falls $R^4$ wie unter $(a_1)$ definiert ist, einen N-terminal mit A und C-terminal mit der NH-Gruppe von Formel I verknüpftem Rest einer Aminosäure bedeutet, die wie unter $(a_2)$ definiert ist, oder
$d_2$) falls $R^4$ wie unter $(a_2)$ oder $(a_3)$ definiert ist, einen N-terminal mit A und C-terminal mit der NH-Gruppe von Formel I verknüpften Rest einer Aminosäure aus der Reihe 2-Thienylalanin, 3-Thienylalanin, Cyclohexylglycin, Phenylglycin, Asparaginsäure, Asparagin, Glutaminsäure, Glutamin und Methioninsulfon bedeutet,
$R^2$ Wasserstoff, $(C_1-C_{10})$-Alkyl, $(C_4-C_7)$-Cycloalkyl, $(C_4-C_7)$-Cycloalkyl-$(C_1-C_4)$-alkyl, $(C_6-C_{14})$-Aryl oder $(C_6-C_{14})$-Aryl-$(C_1-C_4)$-alkyl bedeutet und
$R^3$ Wasserstoff, $(C_1-C_{10})$-Alkyl, $(C_6-C_{14})$-Aryl oder $(C_6-C_{14})$-Aryl-$(C_1-C_4)$-alkyl bedeutet,
sowie deren physiologisch verträglichen Salze.

2. Verbindung der Formel I gemäß Anspruch 1, in welcher A, B und/oder $R^4$ jeweils für den Rest einer L-Aminosäure oder den Rest eines L-Aminosäure-Derivats stehen.

3. Verbindung der Formel I gemäß Anspruch 1 oder 2, in welcher $R^4$ wie im Anspruch 1 unter $(a_1)$ definiert ist.

4. Verbindung der Formel I gemäß Anspruch 1 oder 2, in welcher $R^4$ wie im Anspruch 1 unter $(a_2)$ definiert ist.

5. Verbindung der Formel I gemäß Anspruch 1 oder 2, in welcher $R^4$ wie im Anspruch 1 unter $(a_3)$ definiert ist.

6. Verbindung der Formel I gemäß Anspruch 3, in welcher $R^5$ Wasserstoff oder Methyl bedeuten.

7. Verbindung der Formel I gemäß Anspruch 3 oder 6, in welcher $R^6$ Wasserstoff, Methyl, Ethyl, Propyl, Isopropyl, Butyl, Isobutyl, sec. Butyl, $(C_1-C_5)$-Alkoxycarbonylmethyl, $(C_1-C_5)$-Alkoxycarbonylethyl oder Methylthioethyl bedeuten.

8. Verbindung der Formel I gemäß einem der Ansprüche 3, 6 oder 7, in welcher Ar für Phenoxy, Phenyl, 2-, 3-oder 4-Pyridyl, 4-Imidazolyl, 1-, 3-oder 4-Isochinolyl, 2-, 3-oder 4-Carboxyphenyl, 2-, 3-oder 4-($C_1$-$C_7$)-Alkoxycarbonylphenyl, vorzugsweise 3-Methoxycarbonylphenyl oder 3-tert.-Butoxycarbonylphenyl, 2-, 3-oder 4-Aminophenyl, Mono-oder Di-($C_1$-$C_7$)-alkylaminophenyl, vorzugsweise 3-Methylaminophenyl oder 3-Dimethylaminophenyl, 2-, 3-oder 4-Carbamoylphenyl, 3-Sulfamoylphenyl, 3-Sulfophenyl, Amino-($C_1$-$C_7$)-alkylphenyl, vorzugsweise 2-, 3-oder 4-Aminomethylphenyl, 3-(Methylaminomethyl)-phenyl, 3-(N,N-Dimethylaminomethyl)-phenyl, ($C_1$-$C_7$)-Alkoxycarbonylmethoxyphenyl, vorzugsweise 2-, 3-oder 4-Methoxycarbonylmethoxyphenyl oder 3-tert.-Butoxycarbonylmethoxyphenyl, 2-, 3-oder 4-Carboxy methyloxyphenyl, ($C_1$-$C_7$)-Alkoxyphenyl, vorzugsweise 2-, 3-oder 4-Methoxyphenyl, oder 2-, 3-oder 4-Guanidinomethylphenyl steht.

9. Verbindung der Formel I gemäß Anspruch 4, in welcher $R^4$ für den Rest eines Aminosäureamids mit dem C-Terminus -$NR^7R^8$ steht, worin $R^7$ und $R^8$ gleich oder verschieden sind und Methyl, 10-Aminodecyl, 9-Aminononyl, 8-Aminooctyl, 7-Aminoheptyl, 6-Aminohexyl, 5-Aminopentyl, 4-Aminobutyl oder die N-BOC-oder N-Z-geschützten Derivate der genannten ω-Aminoalkylreste, (2-Pyrimidinyl-amino)-ethyl, 2-Sulfoethyl, 8-Carboxyoctyl, 7-Carboxyheptyl, 6-Carboxyhexyl, 7-Methoxycarbonylheptyl, 2-Pyridylaminoethyl, Phenethyl oder Benzyl, die beide gegebenenfalls im Phenylteil in 2-, 3-oder 4-Position durch Amino, in 2-, 3-oder 4-Position durch Aminomethyl, in 3-Position durch BOC-Aminomethyl oder Guanidinomethyl, in 2-, 3-oder 4-Position durch Carboxy, in 3-Position durch tert.-Butoxycarbonyl, in 2-, 3-oder 4-Postion durch Carboxymethoxy oder in 3-Position durch tert.-Butoxycarbonylmethoxy substituiert sind, 2-, 3-oder 4-Pyridylmethyl, 4-Imidazolylethyl, 3-Indolylethyl, 4-Piperidyl, 4-Piperidyl-($C_1$-$C_4$)-alkyl, 2-Oxo-1-pyrrolidinyl), 2-Oxo-1-pyrrolidinyl)-($C_1$-$C_4$)-alkyl,
vorzugsweise 4-(N-Benzyl)-piperidyl, 3-Morpholinopropyl oder 3-(2-Oxo-1-pyrrolidinyl)-propyl bedeuten oder -$NR^7R^8$ für Morpholino steht.

10. Verbindung der Formel I gemäß Anspruch 5, in welcher $R^4$ für Leu-Asn-$NH_2$, Ile-His-OH, Ile-His-$NH_2$, Ile-Arg-$NH_2$, Ile-Arg-OH, Ile-Arg-$OCH_3$, Ile-Argninol, Ile-Lys-(BOC)-$OCH_3$, Ile-Lys-$OCH_3$, Ile-Lys-OH oder Ile-Lys-$NH_2$ steht.

11. Verbindung der Formel I gemäß einem der Ansprüche 1 -10, in welcher $R^1$ abwesend ist, Wasserstoff bedeutet oder für ($C_1$-$C_{10}$)-Alkyl, Cyclopentyl, Cyclohexyl, Cyclopentyl-($C_1$-$C_{10}$)-alkyl, Cyclohexyl-($C_1$-$C_{10}$)-alkyl, gegebenenfalls substituiertes Phenyl-($C_1$-$C_8$)-alkyl, $H_2N$-($C_1$-$C_{10}$)-Alkyl, HO-($C_1$-$C_{10}$)-Alkyl, ($C_1$-$C_4$)-Alkoxy-($C_1$-$C_{10}$)-alkyl, ($C_1$-$C_4$)-Alkoxycarbonyl-($C_1$-$C_{10}$)-alkyl, Carboxy-($C_1$-$C_{10}$)-alkyl oder ($C_1$-$C_8$)-Alkanoyloxy-($C_1$-$C_{10}$)-alkyl steht.

12. Verbindung der Formel I gemäß einem der Ansprüche 1 -10, in welcher $R^1$ ($C_1$-$C_{11}$)-Alkanoyl, vorzugsweise n-Decanoyl, Formyl, Acetyl, Pivaloyl, Isovaleryl oder Isobutyryl, gegebenenfalls geschütztes Amino-($C_1$-$C_{11}$)-alkanoyl, vorzugsweise 4-Aminobutyryl, 5-Aminopentanoyl, 6-Aminohexanoyl, 4-N-tert.-Butoxycarbonylaminobutyryl, 5-N-tert.Butoxycarbonylaminopentanoyl oder 6-N-tert.-butoxycarbonylaminohexanoyl, Di-($C_1$-$C_7$)-alkylamino-($C_1$-$C_{11}$)-alkanoyl, vorzugsweise Dimethylaminoacetyl, ($C_4$-$C_9$)-Cycloalkanoyl, vorzugsweise Cyclopropanoyl, Cyclobutanoyl, Cyclopentanoyl oder Cyclohexanoyl, ($C_6$-$C_{10}$)-Aryl-($C_1$-$C_{11}$)-alkanoyl, vorzugsweise Phenylacetyl, Phenylpropanoyl oder Phenylbutanoyl, 2-(o,o-Dichloroanilino)-phenylacetyl, 2-(N-Benzyl-o,o-dichloranilino)-phenylacetyl, gegebenenfalls durch Halogen, ($C_1$-$C_7$)-Alkyl, ($C_1$-$C_7$)-Alkoxy, ($C_1$-$C_7$)-Alkoxycarbonyl substituiertes Benzoyl, vorzugsweise 4-Chlorbenzoyl, 4-Methylbenzoyl, 2-Methoxycarbonylbenzoyl oder 4-Methoxybenzoyl, Pyrrolyl-2-carbonyl, Pyridyl-3-carbonyl, Benzylsulfonyl, ($C_1$-$C_{10}$)-Alkoxycarbonyl, vorzugsweise Methoxycarbonyl oder tert.-Butoxy-carbonyl, durch Halogen substituiertes -($C_1$-$C_{10}$)-Alkoxycarbonyl, vorzugsweise 2,2,2-Trichlorethoxycarbonyl oder 1,1-Dimethyl-2,2,2-trichlorethoxycarbonyl, ($C_6$-$C_{14}$)-Aryl-($C_1$-$C_6$)-alkoxycarbonyl, vorzugsweise Benzyloxycarbonyl oder 9-Fluorenylmethylcarbonyl, (2S)-trans-4-Hydroxy-1-acetylpyrrolidinyl-2-carbonyl, α-Methoxycarbonyl-α-benzyloxycarbonylaminoacetyl, N-Acetyl-(2S,3aS,6aS)-octahydrocyclopenta[b]pyrrol-2-carbonyl oder N-Acetyl-(2R,3aR,6aR)-octahydrocyclopenta[b]pyrrol-2-carbonyl bedeutet.

13. Verbindung der Formel I gemäß einem der Ansprüche 1, 2, 4, 9, 11 oder 12, in welcher $R^4$ einen Rest einer Aminosäure aus der Reihe Phenylalanin, Histidin, Tyrosin, Tryptophan, Methionin, Leucin, Isoleucin, Asparagin, Asparaginsäure, β-2-Thienylalanin, β-3-Thienylalanin, 4-Chlorphenylalanin, Methioninsulfon, Methioninsulfoxid, 2-Pyridylalanin, 3-Pyridylalanin, Cyclohexylalanin, Cyclohexylglycin, im-Methylhistidin, O-Methyltyrosin, O-Benzyltyrosin, O-tert.-Butyltyrosin, Phenylglycin, 1-Naphthylalanin, 2-Naphtylalanin, 4-Nitrophenylalanin, Norleucin, 1,2,3,4-Tetrahydroisochinolin-3-carbonsäure, Homophenylalanin, 2-Amino-4-(2-thienyl)-buttersäure, (Z)-Dehydrophenylalanin oder (E)-Dehydrophenylalanin bedeutet dessen C-Terminus wie im Anspruch 1 definiert vorliegt.

14. Verbindung der Formel I gemäß einem der Ansprüche 1 -3, 6 -8, 11 oder 12 in welcher B einen N-terminal mit A und C-terminal mit der NH-Gruppe von Formel I verknüpften Rest einer Aminosäure bedeutet, die wie die Aminosäuren in Anspruch 13 definiert ist.

15. Verbindung der Formel I gemäß einem der Ansprüche 1 -14, in welcher A einen N-terminal mit $R^1$ und C-terminal mit B verknüpften Rest einere Aminosäure bedeutet, die wie die Aminosäuren im Anspruch 13 definiert ist, (2S)-O-Benzyl-3-phenyl-propanoyl oder (2R,S)-2-Benzyl-4-oxo-6,6-dimethyl-6-heptanoyl bedeutet.

16. Verbindung der Formel I gemäß einem der Ansprüche 1 -15, in welcher $R^2$ Cyclohexylmethyl, Benzyl oder Isobutyl bedeutet.

17. Verbindung der Formel I gemäß einem der Ansprüche 1 -16, in welcher $R^3$ Wasserstoff, Ethyl, sec.-Butyl, Isobutyl, Benzyl, Phenethyl, oder 3-Phenylpropyl bedeutet.

18. Verfahren zur Herstellung einer Verbindung der Formel I gemäß einem der Ansprüche 1 -17, dadurch gekennzeichnet, daß man ein Fragment mit endständiger Carboxylgruppe oder reaktives Derivat mit einem entsprechenden Fragment mit freier Aminogruppe kuppelt, gegebenenfalls zum Schutz weiterer funktioneller Gruppen (eine) temporär eingeführte Schutzgruppe(n) abspaltet und die so erhaltene Verbindung gegebenenfalls in ihr physiologisch verträgliches Salz überführt.

19. Verbindung gemäß einem der Ansprüche 1-17 zur Anwendung als Heilmittel.

20. Verbindung gemäß einem der Ansprüche 1-17 zur Anwendung als Heilmittel bei der Behandlung des Bluthochdrucks.

21. Pharmazeutisches Mittel enthaltend eine Verbindung gemäß einem der Ansprüche 1 - 17.

Patentansprüche für folgende Vertragsstaaten: GR, ES und AT:

1. Verfahren zur Herstellung einer Verbindung der Formel I

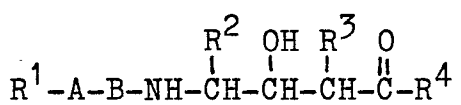

(I)

in welcher

$R^4$ $a_1$) einen Rest der Formel II oder III bedeutet,

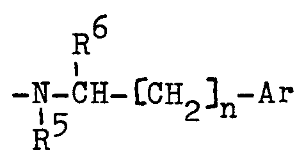

(II)

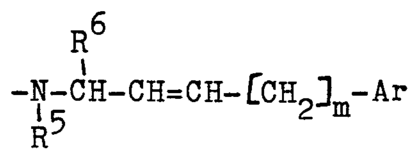

(III)

worin $R^5$ für Wasserstoff oder $(C_1$-$C_7)$-Alkyl und $R^6$ für Wasserstoff, $(C_1$-$C_7)$-Alkyl oder durch Hydroxy, $(C_1$-$C_5)$-Alkoxy, $(C_1$-$C_5)$-Alkylthio, Carboxy, $(C_1$-$C_5)$-Alkoxycarbonyl, Cl, Br, $(C_1$-$C_5)$-Alkylamino, Di-$(C_1$-$C_5)$-alkylamino, $(C_1$-$C_5)$-Alkoxycarbonylamino oder $(C_1$-$C_{15})$-Aralkyloxycarbonylamino monosubstituiertes $(C_1$-$C_7)$-Alkyl stehen,

m = 0, 1, 2, 3 oder 4 ist,

n = 1, 2, 3, 4, 5, oder 6 ist,

die Doppelbindung im Rest der Formel III im Falle n = 0 vorzugsweise die E-Konfiguration, andernfalls vorzugsweise die Z-Konfiguration aufweist und

Ar für $(C_6$-$C_{14})$-Aryl, das gegebenenfalls durch einen oder zwei gleiche oder verschiedene Reste aus der Reihe F, Cl, Br, J, Hydroxy, $(C_1$-$C_7)$-Alkoxy, $(C_1$-$C_7)$-Alkyl, $(C_1$-$C_7)$-Alkoxycarbonyl, Amino, $(C_1$-$C_7)$-Alkylamino, Di-$(C_1$-$C_7)$-Alkylamino, Carboxy, Carboxymethyloxy, Amino-$(C_1$-$C_7)$-alkyl, $(C_1$-$C_7)$-Alkylamino-$(C_1$-$C_7)$-alkyl, Di-$(C_1$-$C_7)$-alkylamino-$(C_1$-$C_7)$-alkyl, $(C_1$-$C_7)$-Alkoxycarbonylmethoxy, Carbamoyl, Sulfamoyl, $(C_1$-$C_7)$-Alkoxysulfonyl, Sulfo und Guanidinomethyl substituiert ist, oder für den Rest eines 5-oder 6-gliedrigen monocyclischen oder 9-oder 10-gliedrigen bicyclischen Heteroaromaten mit mindestens 1 C-Atom, 1 -3 N-Atomen und/oder

1 S-oder O-Atom als Ringglieder steht, wobei Ar auch über -O-gebunden sein kann,

$a_2$) einen über eine Peptidbindung verknüpften Rest einer Aminosäure aus der Reihe Phenylalanin, Histidin, Tyrosin, Tryptophan, Methionin, Leucin, Isoleucin, Asparagin, Asparaginsäure, β-2-Thienylalanin, β-3-Thienylalanin, 4-Chlorphenylalanin, Methioninsulfon, Methioninsulfoxid, 2-Pyridylalanin, 3-Pyridylalanin, Cyclohexylalanin, Cyclohexylglycin, im-Methylhistidin, O-Methyltyrosin, O-Benzyltyrosin, O-tert.-Butyltyrosin, Phenylglycin, 1-Naphthylalanin, 2-Naphthylalanin, 4-Nitrophenylalanin, 2-Fluorphenylalanin, 3-Fluorphenylalanin, 4-Fluorphenylalanin, Norleucin, 1,2,3,4-Tetrahydroisochinolin-3-carbonsäure, Homophenylalanin, DOPA, O-Dimethyldopa, 2-Amino-4-(2-thienyl)-buttersäure, 2-Amino-4-(3-thienyl)-buttersäure, 3-(2-Thienyl)-serin, (Z)-Dehydrophenylalanin und (E)-Dehydrophenylalanin, dessen C-terminale Carboxylgruppe frei, zur $CH_2OH$ reduziert oder

als Amid, welches einen C-Terminus der Formel $-NR^7R^8$ aufweist, vorliegt, worin $R^7$ und $R^8$ gleich oder verschieden sind und für

Wasserstoff,

$(C_1-C_{10})$-Alkyl, das gegebenenfalls durch Amino, $(C_1-C_5)$-Alkoxycarbonylamino, $(C_7-C_{15})$-Aralkylcarbonylamino, Heteroarylamino, worin der Rest des Heteroaromaten wie oben unter $(a_1)$ definiert ist, Hydroxy, $(C_1-C_5)$-Alkoxy, Sulfo, Carboxy oder $(C_1-C_5)$-Alkoxycarbonyl monosubstituiert ist,

$(C_6-C_{14})$-Aryl-$(C_1-C_4)$-alkyl, das gegebenenfalls im Arylteil durch Amino, Carboxy, $(C_1-C_5)$-Alkoxycarbonyl, Amino-$(C_1-C_5)$-alkyl, $(C_1-C_5)$-Alkoxycarbonylamino-$(C_1-C_5)$-alkyl, $(C_7-C_{15})$-Aralkoxycarbonylamino-$(C_1-C_5)$-alkyl, $(C_1-C_5)$-Alkoxycarbonyl-$(C_1-C_5)$-alkoxy, Carboxy-$(C_1-C_5)$-alkoxy oder Guanido-$(C_1-C_5)$-alkoxy monosubstituiert ist, oder

Heteroaryl-$(C_1-C_4)$-alkyl, wobei der Rest des Heteroaromaten wie Ar unter $(a_1)$ definiert ist, oder für einen Rest der Formel IV steht,

$$-\ CH_2{}_p-\widehat{Q[CH_2]_q} \qquad (IV)$$

worin p = 1, 2, 3 oder 4 ist, q = 4 oder 5 ist und Q N oder CH bedeutet oder p = 0 ist, q = 4 oder 5 ist und Q CH bedeutet und worin eine $CH_2$-Gruppe im Ring durch NH, O, S, CO, N-Bzl, N-Z oder N-BOC ersetzt sein kann, oder

$a_3$) einen über eine Peptidbindung verknüpften Rest eines Dipeptids aus der Reihe Leu-Asn, Ile-His, Ile-Arg und Ile-Lys bedeutet, dessen C-Terminus als freie COOH-Gruppe, die gegebenenfalls zur $CH_2OH$-Gruppe reduziert ist, als $CONH_2$ oder als $COOR^9$ mit $R^9 = (C_1-C_7)$-Alkyl vorliegt und worin die ε-Aminofunktion des Lys gegebenenfalls durch $(C_1-C_5)$-Alkoxycarbonyl oder $(C_7-C_{15})$-Aralkyloxycarbonyl geschützt ist;

$R^1$ $b_1$) abwesend ist oder Wasserstoff bedeutet,

$b_2$) $(C_1-C_{20})$-Alkyl, das gegebenenfalls durch ein, zwei oder drei gleiche oder verschiedene Reste aus der Reihe Hydroxy, $(C_1-C_7)$-Alkoxy, $(C_1-C_7)$-Alkanoyloxy, Carboxy, $(C_1-C_7)$-Alkoxycarbonyl, $(C_1-C_8)$-Alkanoyloxy, Cl, Br, Amino, $(C_1-C_7)$-Alkylamino, Di-$(C_1-C_7)$-alkylamino, $(C_1-C_5)$-Alkoxycarbonylamino oder $(C_7-C_{11})$-Aralkyloxycarbonylamino substituiert ist,

$(C_3-C_8)$-Cycloalkyl,

$(C_3-C_8)$-Cycloalkyl-$(C_1-C_{10})$alkyl oder

$(C_6-C_{14})$-Aryl-$(C_1-C_5)$-alkyl, das im Arylteil gegebenenfalls durch einen oder zwei gleiche oder verschiedene Reste aus der Reihe F, Cl, Br, Hydroxy, $(C_1-C_7)$-Alkoxy, $(C_1-C_7)$-Alkyl, $(C_1-C_7)$-Alkoxycarbonyl, Amino oder Trifluormethyl substituiert ist, bedeutet oder

$b_3$) einen Rest der Formel V bedeutet,

$R^a$ -W-(V)

worin W für -CO-, -O-CO-, $-SO_2$-oder -NH-CO-steht und $R^a$ Wasserstoff, $(C_1-C_{10})$-Alkyl, das gegebenenfalls ein-oder zweifach ungesättigt ist und das gegebenenfalls durch bis zu 3 gleiche oder verschiedene Rest aus der Reihe Hydroxy, $(C_1-C_7)$-Alkoxy, $(C_1-C_7)$-Alkanoyloxy, Carboxy, $(C_1-C_7)$-Alkoxycarbonyl, Cl, Br, Amino, $(C_1-C_7)$-Alkylamino, Di-$(C_1-C_7)$-Alkyl amino, $(C_1-C_5)$-Alkoxycarbonylamino, $(C_7-C_{15})$-Aralkoxycarbonylamino oder 9-Fluorenylmethyloxycarbonylamino monosubstituiert ist,

$(C_3-C_8)$-Cycloalkyl,

$(C_3-C_8)$-Cycloalkyl-$(C_1-C_6)$alkyl,

$(C_6-C_{14})$-Aryl, das gegebenenfalls durch einen oder zwei gleiche oder verschiedene Reste aus der Reihe F, Cl, Br, J, Hydroxy, $(C_1-C_7)$-Alkoxy, $(C_1-C_7)$-Alkyl, $(C_1-C_7)$-Alkoxycarbonyl, Amino, gegebenenfalls mit bis zu 2 Halogen substituiertes Anilino und Trifluormethyl substituiert ist,

$(C_6-C_{14})$-Aryl-$(C_1-C_6)$-alkyl, worin der Arylteil gegebenenfalls wie vorstehend bei Aryl definiert ist, Heteroaryl

oder Heteroaryl-($C_1$-$C_6$)-alkyl, wobei der Rest des Heteroaromaten jeweils wie Ar unter ($a_1$) definiert ist, bedeutet, oder

$b_4$) einen Rest aus der Reihe (2S)-trans-4-Hydroxy-1-acyl-pyrrolidinyl-2-carbonyl, 5-Pyrrolidon-2S-carbonyl, α-Methoxycarbonyl-α-acylacetyl, 1-Acyl-(2R, 3aR, 6aR)-octahydro-cyclopenta[b]pyrrol-2-carbonyl und 1-Acyl-(2S,3aS,6aS)-octahydro-cyclo-penta[b]pyrrol-2-carbonyl bedeutet, wobei Acyl die Bedeutung ($C_1$-$C_6$)-Alkanoyl, ($C_1$-$C_5$)-Alkoxycarbonyl oder ($C_7$-$C_{15}$)-Alkoxycarbonyl oder ($C_7$-$C_{15}$)-Aralkyloxycarbonyl hat,

A $c_1$) einen Rest der Formeln VI oder VII bedeutet,

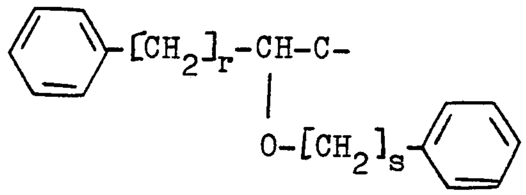

(VI)

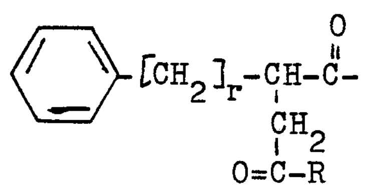

(VII)

in welchen

r = 1, 2 oder 3 ist,

s = 1, 2 oder 3 ist und

R ($C_1$-$C_7$)-Alkyl bedeutet, oder

$c_2$) einen N-terminal mit $R^1$ und C-terminal mit B verknüpften Rest einer Aminosäure bedeutet, die wie unter ($a_2$) definiert ist,

B $d_1$) falls $R^4$ wie unter ($a_1$) definiert ist, einen N-terminal mit A und C-terminal mit der NH-Gruppe von Formel I verknüpftem Rest einer Aminosäure bedeutet, die wie unter ($a_2$) definiert ist, oder

$d_2$) falls $R^4$ wie unter ($a_2$) oder ($a_3$) definiert ist, einen N-terminal mit A und C-terminal mit der NH-Gruppe von Formel I verknüpften Rest einer Aminosäure aus der Reihe 2-Thienylalanin, 3-Thienylalanin, Cyclohexylglycin, Phenylglycin, Asparaginsäure, Asparagin, Glutaminsäure, Glutamin und Methioninsulfon bedeutet,

$R^2$ Wasserstoff, ($C_1$-$C_{10}$)-Alkyl, ($C_4$-$C_7$)-Cycloalkyl, ($C_4$-$C_7$)-Cycloalkyl-($C_1$-$C_4$)-alkyl, ($C_6$-$C_{14}$)-Aryl oder ($C_6$-$C_{14}$)-Aryl-($C_1$-$C_4$)-alkyl bedeutet und

$R^3$ Wasserstoff, ($C_1$-$C_{10}$)-Alkyl, ($C_6$-$C_{14}$)-Aryl oder ($C_6$-$C_{14}$)-Aryl-($C_1$-$C_4$)-alkyl bedeutet,

sowie deren physiologisch verträglichen Salze, dadurch gekennzeichnet, daß man ein Fragment mit endständiger Carboxylgruppe oder reaktives Derivat mit einem entsprechenden Fragment mit freier Aminogruppe kuppelt, gegebenenfalls zum Schutz weiterer funktioneller Gruppen (eine) temporär eingeführte Schutzgruppe(n) abspaltet und die so erhaltene Verbindung gegebenenfalls in ihr physiologisch verträgliches Salz überführt.

2. Verfahren gemäß Anspruch 1, dadurch gekennzeichnet, daß man eine Verbindung der Formel I herstellt, in welcher A, B und/oder $R^4$ jeweils für den Rest einer L-Aminosäure oder den Rest eines L-Aminosäure-Derivats stehen.

3. Verfahren gemäß Anspruch 1 oder 2, dadurch gekennzeichnet, daß man eine Verbindung der Formel I herstellt, in welcher $R^4$ wie im Anspruch 1 unter ($a_1$) definiert ist.

4. Verfahren gemäß Anspruch 1 oder 2, dadurch gekennzeichnet, daß man eine Verbindung der Formel I herstellt, in welcher $R^4$ wie im Anspruch 1 unter ($a_2$) definiert ist.

5. Verfahren gemäß Anspruch 1 oder 2, dadurch gekennzeichnet, daß man eine Verbindung der Formel I herstellt, in welcher $R^4$ wie im Anspruch 1 unter ($a_3$) definiert ist.

6. Verfahren gemäß Anspruch 3, dadurch gekennzeichnet, daß man eine Verbindung der Formel I herstellt, in welcher $R^5$ Wasserstoff oder Methyl bedeuten.

7. Verfahren gemäß Anspruch 3 oder 6, dadurch gekennzeichnet, daß man eine Verbindung der Formel I herstellt, in welcher $R^6$ Wasserstoff, Methyl, Ethyl, Propyl, Isopropyl, Butyl, Isobutyl, sec. Butyl, ($C_1$-$C_5$)-Alkoxycarbonylmethyl, ($C_1$-$C_5$)-Alkoxycarbonylethyl oder Methylthioethyl bedeuten.

8. Verfahren gemäß einem der Ansprüche 3, 6 oder 7, dadurch gekennzeichnet, daß man eine Verbindung der Formel I herstellt, in welcher Ar für Phenoxy, Phenyl, 2-, 3-oder 4-Pyridyl, 4-Imidazolyl, 1-, 3-oder 4-Isochinolyl, 2-, 3-oder 4-Carboxyphenyl, 2-, 3-oder 4-($C_1$-$C_7$)-Alkoxycarbonylphenyl, vorzugsweise 3-Methoxycarbonylphenyl oder 3-tert.-Butoxycarbonylphenyl, 2-, 3-oder 4-Aminophenyl, Mono-oder Di-($C_1$-$C_7$)-alkylaminophenyl, vorzugsweise 3-Methylaminophenyl oder 3-Dimethylaminophenyl, 2-, 3-oder 4-Carbamoylphenyl, 3-Sulfamoylphenyl, 3-Sulfophenyl, Amino-($C_1$-$C_7$)-alkylphenyl, vorzugsweise 2-, 3-oder 4-Aminomethylphenyl, 3-(Methylaminomethyl)-phenyl, 3-(N,N-Dimethylaminomethyl)-phenyl, ($C_1$-$C_7$)-Alkoxycarbonylmethoxyphenyl, vorzugsweise 2-, 3-oder 4-Methoxycarbonylmethoxyphenyl oder 3-tert.-Butoxycarbonylmethoxyphenyl, 2-, 3-oder 4-Carboxymethyloxyphenyl, ($C_1$-$C_7$)-Alkoxyphenyl, vorzugsweise 2-, 3-oder 4-Methoxyphenyl, oder 2-, 3-oder 4-Guanidinomethylphenyl steht.

9. Verfahren gemäß Anspruch 4, dadurch gekennzeichnet, daß man eine Verbindung der Formel I herstellt, in welcher $R^4$ für den Rest eines Aminosäureamids mit dem C-Terminus -$NR^7R^8$ steht, worin $R^7$ und $R^8$ gleich oder verschieden sind und Methyl, 10-Aminodecyl, 9-Aminononyl, 8-Aminooctyl, 7-Aminoheptyl, 6-Aminohexyl, 5-Aminopentyl, 4-Aminobutyl oder die N-BOC-oder N-Z-geschützten Derivate der genannten $\omega$-Aminoalkylreste, (2-Pyrimidinyl-amino)-ethyl, 2-Sulfoethyl, 8-Carboxyoctyl, 7-Carboxyheptyl, 6-Carboxyhexyl, 7-Methoxycarbonylheptyl, 2-Pyridylaminoethyl, Phenethyl oder Benzyl, die beide gegebenenfalls im Phenylteil in 2-, 3-oder 4-Position durch Amino, in 2-, 3-oder 4-Position durch Aminomethyl, in 3-Position durch BOC-Aminomethyl oder Guanidinomethyl, in 2-, 3-oder 4-Position durch Carboxy, in 3-Position durch tert.-Butoxycarbonyl, in 2-, 3-oder 4-Postion durch Carboxymethoxy oder in 3-Position durch tert.-Butoxycarbonylmethoxy substituiert sind, 2-, 3-oder 4-Pyridylmethyl, 4-Imidazolylethyl, 3-Indolylethyl, 4-Piperidyl, 4-Piperidyl-($C_1$-$C_4$)-alkyl, 2-Oxo-1-pyrrolidinyl), 2-Oxo-1-pyrrolidinyl)-($C_1$-$C_4$)-alkyl, vorzugsweise 4-(N-Benzyl)-piperidyl, 3-Morpholinopropyl oder 3-(2-Oxo-1-pyrrolidinyl)-propyl bedeuten oder -$NR^7R^8$ für Morpholino steht.

10. Verfahren gemäß Anspruch 5, dadurch gekennzeichnet, daß man eine Verbindung der Formel I herstellt, in welcher $R^4$ für Leu-Asn-$NH_2$, Ile-His-OH, Ile-His-$NH_2$, Ile-Arg-$NH_2$, Ile-Arg-OH, Ile-Arg-$OCH_3$, Ile-Arginol, Ile-Lys-(BOC)-$OCH_3$, Ile-Lys-$OCH_3$, Ile-Lys-OH oder Ile-Lys-$NH_2$ steht.

11. Verfahren gemäß einem der Ansprüche 1 -10, dadurch gekennzeichnet, daß man eine Verbindung der Formel I herstellt, in welcher $R^1$ abwesend ist, Wasserstoff bedeutet oder für ($C_1$-$C_{10}$)-Alkyl, Cyclopentyl, Cyclohexyl, Cyclopentyl-($C_1$-$C_{10}$)-alkyl, Cyclohexyl-($C_1$-$C_{10}$)-alkyl, gegebenenfalls substituiertes Phenyl-($C_1$-$C_8$)-alkyl, $H_2N$-($C_1$-$C_{10}$)-Alkyl, HO-($C_1$-$C_{10}$)-Alkyl, ($C_1$-$C_4$)-Alkoxy-($C_1$-$C_{10}$)-alkyl, ($C_1$-$C_4$)-Alkoxycarbonyl-($C_1$-$C_{10}$)-alkyl, Carboxy-($C_1$-$C_{10}$)-alkyl oder ($C_1$-$C_8$)-Alkanoyloxy-($C_1$-$C_{10}$)-alkyl steht.

12. Verfahren gemäß einem der Ansprüche 1 -10, dadurch gekennzeichnet, daß man eine Verbindung der Formel I herstellt, in welcher $R^1$ ($C_1$-$C_{11}$)-Alkanoyl, vorzugsweise n-Decanoyl, Formyl, Acetyl, Pivaloyl, Isovaleryl oder Isobutyryl, gegebenenfalls geschütztes Amino-($C_1$-$C_{11}$)-alkanoyl, vorzugsweise 4-Aminobutyryl, 5-Aminopentanoyl, 6-Aminohexanoyl, 4-N-tert.-Butoxycarbonylaminobutyryl, 5-N-tert.Butoxycarbonylaminopentanoyl oder 6-N-tert.-butoxycarbonylaminohexanoyl, Di-($C_1$-$C_7$)-alkylamino-($C_1$-$C_{11}$)-alkanoyl, vorzugsweise Dimethyl aminoacetyl, ($C_4$-$C_9$)-Cycloalkanoyl, vorzugsweise Cyclo-propanoyl, Cyclobutanoyl, Cyclopentanoyl oder Cyclohexanoyl, ($C_6$-$C_{10}$)-Aryl-($C_1$-$C_{11}$)-alkanoyl, vorzugsweise Phenylacetyl, Phenylpropanoyl oder Phenylbutanoyl, 2-(o,o-Dichloroanilino)-phenylacetyl, 2-(N-Benzyl-o,o-dichloranilino)-phenylacetyl, gegebenenfalls durch Halogen, ($C_1$-$C_7$)-Alkyl, ($C_1$-$C_7$)-Alkoxy, ($C_1$-$C_7$)-Alkoxycarbonyl substituiertes Benzoyl, vorzugsweise 4-Chlorbenzoyl, 4-Methylbenzoyl, 2-Methoxycarbonylbenzoyl oder 4-Methoxybenzoyl, Pyrrolyl-2-carbonyl, Pyridyl-3-carbonyl, Benzylsulfonyl, ($C_1$-$C_{10}$)-Alkoxycarbonyl, vorzugsweise Methoxycarbonyl oder tert.-Butoxy-carbonyl, durch Halogen substituiertes ($C_1$-$C_{10}$)-Alkoxycarbonyl, vorzugsweise 2,2,2-Trichlorethoxycarbonyl oder 1,1-Dimethyl-2,2,2-trichlorethoxycarbonyl, ($C_6$-$C_{14}$)-Aryl-($C_1$-$C_6$)-alkoxycarbonyl, vorzugsweise Benzyloxycarbonyl oder 9-Fluorenylmethylcarbonyl, (2S)-trans-4-Hydroxy-1-acetylpyrrolidinyl-2-carbonyl, $\alpha$-Methoxycarbonyl-$\alpha$-benzyloxycarbonylaminoacetyl, N-Acetyl-(2S,3aS,6aS)-octahydrocyclopenta[b]pyrrol-2-carbonyl oder N-Acetyl-(2R,3aR,6aR)-octahydrocyclopenta[b]pyrrol-2-carbonyl bedeutet.

13. Verfahren gemäß einem der Ansprüche 1, 2, 4, 9, 11 oder 12, dadurch gekennzeichnet, daß man eine Verbindung der Formel I herstellt, in welcher $R^4$ einen Rest einer Aminosäure aus der Reihe Phenylalanin, Histidin, Tyrosin, Tryptophan, Methionin, Leucin, Isoleucin, Asparagin, Asparaginsäure, $\beta$-2-Thienylalanin, $\beta$-3-Thienylalanin, 4-Chlorphenylalanin, Methioninsulfon, Methioninsulfoxid, 2-Pyridylalanin, 3-Pyridylalanin, Cyclohexylalanin, Cyclohexylglycin, im-Methylhistidin, O-Methyltyrosin, O-Benzyltyrosin, O-tert.-Butyltyrosin, Phenylglycin, 1-Naphthylalanin, 2-Naphtylalanin, 4-Nitrophenylalanin, Norleucin, 1,2,3,4-Tetrahydroisochinolin-3-carbonsäure, Homophenylalanin, 2-Amino-4-(2-thienyl)-buttersäure, (Z)-Dehydrophenyl alanin oder (E)-Dehydrophenylalanin bedeutet dessen C-Terminus wie im Anspruch 1 definiert vorliegt.

14. Verfahren gemäß einem der Ansprüche 1 -3, 6 -8, 11 oder 12, dadurch gekennzeichnet, daß man eine Verbindung der Formel I herstellt, in welcher B einen N-terminal mit A und C-terminal mit der NH-Gruppe von Formel I verknüpften Rest einer Aminosäure bedeutet, die wie die Aminosäuren in Anspruch 13 definiert ist.

15. Verfahren gemäß einen der Ansprüche 1 -14, dadurch gekennzeichnet, daß man eine Verbindung der Formel I herstellt, in welcher A einen N-terminal mit $R^1$ und C-terminal mit B verknüpften Rest einere Aminosäure bedeutet, die wie die Aminosäuren im Anspruch 13 definiert ist, (2S)-O-Benzyl-3-phenylpropanoyl oder (2R,S)-2-Benzyl-4oxo-6,6-dimethyl-6-heptanoyl bedeutet.

16. Verfahren gemäß einem der Ansprüche 1 -15, dadurch gekennzeichnet, daß man eine Verbindung der Formel I herstellt, in welcher $R^2$ Cyclohexylmethyl, Benzyl oder Isobutyl bedeutet.

17. Verfahren gemäß einem der Ansprüche 1 - 16, dadurch gekennzeichnet, daß man eine Verbindung der Formel I herstellt, in welcher $R^3$ Wasserstoff, Ethyl, sec.-Butyl, Isobutyl, Benzyl, Phenethyl, oder 3-Phenylpropyl bedeutet.

18. Verfahren zur Herstellung eines pharmazeutischen Mittels, enthaltend eine Verbindung der Formel I gemäß einem der Ansprüche 1 -17, dadurch gekennzeichnet, daß man diese und einen Träger in eine geeignete Darreichungsform bringt.